# EUROPEAN PATENT SPECIFICATION

(11) **EP 1 609 495 B1**
(45) Date of publication and mention of the grant of the patent: **20.07.2011**
(21) Application number: 05012620.0
(22) Date of filing: 13.06.2005
(51) Int. Cl.: A61M 1/36, A61M 1/02

(54) **Bag assembly for the separation of a composite liquid**
Beutelanordnung zur Trennung einer zusammengesetzten Flüssigkeit
Ensemble poche permettant de séparer un liquide composite

(30) Priority: 22.06.2004 US 582385 P
(43) Date of publication of application: 28.12.2005
(73) Proprietor: CaridianBCT, Inc., Lakewood, CO 80215 (US)
(72) Inventor: Dolecek, Victor, Centennial Colorado 80111 (US); Hake, Chuck, Arvada, CO 80003 (US); Hermann, Kristina, Arvada, CO 80005 (US); Ladtkow, James, Broomfield, CO 80020 (US); Martinez, Michael, Golden, CO 80403 (US); Holmes, Brian M., Lakewood, CO 80227 (US); Fiore, Robert, Evergreen, CO 80439 (US); Dahlberg, Johan, 18330 Taby (SE); Hagström, Johan-Petter, 12940 Hagersten (SE); Nordgren, Peter, 11647 Stockholm (SE)
(74) Representative: Roberts, Mark Peter

(56) References cited:
- WO-A-2004/018021
- US-A- 5 100 564
- US-A- 5 549 540

## Description

The present invention concerns a bag assembly for the separation of a composite liquid into at least two components.

The bag assembly of the invention is particularly appropriate for carrying out various methods for the separation of biological fluids comprising an aqueous component and one or more cellular components. Such methods include, for example: extracting a platelet component from several concentrated platelet components pooled together; extracting a plasma component and a red blood cell component from a volume of filtered blood obtained by flowing a volume of whole blood through a filter, removing platelets and white blood cells therefrom; extracting a plasma component, a platelet component and a red blood cell component from a volume of filtered blood obtained by flowing a volume of whole blood through a filter, removing white blood cells therefrom; extracting a plasma component and a cellular component (including platelets, white blood cells, and red blood cells) from a volume of whole blood, the cellular component being subsequently filtered so as to remove platelets and white blood cells therefrom; extracting a plasma component, a platelet component, and a red blood cell component from a volume of whole blood, the white blood cells being subsequently removed by filtration from the platelet component and the red blood cell component.

The international patent application WO 2004/018021 describes a machine for the separation of blood components and a bag set designed for cooperating with this centrifuge machine.

The bag set comprises:
- a separation bag having an annular or a partially annular chamber delimited by an outer circular edge and an inner circular edge;
- two or three product bags that are connected by tubes to the separation bag, at the inner edge thereof;
- an optional secondary product bag connected by a tube to one of the product bags;
- an optional collection bag connected by a tube to the separation bag, at the inner edge thereof; and
- one or more white blood cell filter(s) optionally connected to one or more tubes.
The bags and the tubes of this bag set are made of flexible plastic material. They are pre-connected and the bag set is sterilized before use.

The blood product separation machine described in the international patent application WO 2004/018021 comprises:
- a centrifuge having a rotor that is coupled to a motor adapted to rotate the rotor at such rotation speeds suitable for the separation of blood components. The rotor comprises a shaft having a hollow upper portion defining a container for receiving blood product bags, and a turntable connected to the upper part of the container. The turntable includes an annular chamber that can be closed by a lid and define therewith a separation chamber designed for receiving a separation bag. Pinch valves are mounted on the rotor for selectively closing and opening the tubes connecting the product bags to the separation bag.
- a hydraulic system for pumping a hydraulic liquid in and out an annular hydraulic chamber defined within the separation chamber by a flexible annular diaphragm partially lining the turntable. When the hydraulic liquid is pumped into the hydraulic chamber, the hydraulic chamber expands within the separation chamber, and, if a separation bag full of liquid is present therein, causes a transfer of the content of the separation bag into one of the products bags.
- a control system for essentially controlling the motor coupled to the rotor, i.e. the rotation speed of the motor, the hydraulic system, and the pinch valves mounted on the rotor.

The manipulation of a set of bags of the above mentioned type, which can comprise up to six flexible bags interconnected by flexible tubes of variable lengths, both during the manufacture of the bag set and during its setting in place in a separation machine and its withdrawal therefrom may sometimes be cumbersome. Also, if it happens during a steam sterilization process that a tube kinks, the inner walls of the tube that are in contact usually weld together at the kink and the whole set has to be subsequently discarded.

US 5,549,540 is directed to an insert for a multiple bucket type centrifuge. The insert has two concavely shaped sections which are joined together at their back and includes a block for stability. The inserts include pins to receive blood bags to be centrifuged.

An aim of the invention is to design a bag assembly that is both easy to manufacture and to manipulate. According to the invention, a bag assembly for the separation of a composite liquid into at least two components, comprises:
- a set of bags comprising:
   - a separation bag;
   - at least one product bag, and
   - a product tube connecting the separation bag to the at least one product bag;
- a bag-assembling device for assembling the set of bags comprising:
   - a support member;
   - a mounting means, connected to the support member, for removably mounting the assembling device in a rotor of a centrifuge having a central cavity for containing the at least one product bag; and
   - a first securing means connected to the support member for releasably securing the at least one product bag to the support member,
      wherein the mounting means and the first securing means are so arranged with respect to each other that the at least one product bag occupies a determined position in the central cavity of a rotor when the assembling device in mounted therein.

Additional or alternative features of the invention are as follows:
- The bag assembly comprises a plurality of products bags, and the first securing means is adapted to releasably secure the product bags to the support member so that they form a compact stack.
- The first securing means is adapted to secure at least one product bag to the support member by a peripheral edge thereof.
- The at least one product bag is substantially rectangular and the first securing means is adapted to secure the product bag to the support member by an upper edge thereof.
- The first securing means is adapted to secure at least one product bag to the support member by at least two areas thereof.
- The first securing means comprises at least two spaced apart protruding elements for engaging two holes in a peripheral area of at least one product bag.
- The first securing means further comprises at least two straps, and each strap has a first end connected to the support member and a second end connectable to one of the protruding elements so as to prevent a bag engaged on the protruding element from escaping therefrom.
- The support member comprises an elongated body having two opposite lateral sides, the first and second protruding elements comprise two rods extending substantially perpendicularly from one side of the body of the support member, and each rod has a tip fitted with a retaining extension for preventing a bag engaged on the rods from escaping therefrom.
- The support member comprises a first and a second U-shaped elements having a straight flat base, the first and a second U-shaped elements are releasably connectable so that their respective straight flat base are substantially parallel and define an oblong gap therebetween, and the two spaced apart protruding elements comprise two substantially parallel rods that are connected to the straight flat base of the first U-shaped elements and extend across the oblong gap to the straight flat base of the second U-shaped elements.
- The support member comprises a first and second flaps hinged together and the at least two spaced apart protruding elements extend from an outside surface of the flaps.
- The bag assembly comprises a plurality of product bags and the first securing means comprises four spaced apart protruding elements, two protruding elements extending from the first flap and two protruding elements extending from the second flap.
- The first securing means comprises at least one a jaw for releasably grasping at least one product bag along a portion of a peripheral edge thereof.
- The bag assembly comprises a plurality of products bags, the support member comprises an elongated body having two lateral sides, and the first securing means comprises two jaws respectively hinged to either side of the elongated body.
- The mounting means is connected to the support member so that the support member intersects a rotation axis of the rotor when the bag-assembling device is mounted in the rotor.
- The support member comprises an elongated portion having two lateral ends, and the mounting means comprises two extensions extending at both ends of the elongated portion of the support member, wherein the two extensions are adapted to engage two corresponding recesses in a wall of a central cavity of a rotor so that the support member extends across the cavity of the rotor when the lateral extensions are engaged in the recesses thereof.
- The support member comprises an elongated body having two opposite lateral sides, and the mounting means comprises a first and second protruding elements extending from one side of the body of the support member, wherein the protruding elements have tips adapted to engage two recesses in a portion of a wall of a central cavity of a rotor so that the support member extends substantially along the portion of wall when the protruding elements are engaged in the recesses thereof.
- The mounting means and the first securing means are so arranged with respect to each other that the at least one product bag hangs substantially in an upright position when the bag-assembling device is mounted in the rotor of a centrifuge.
- The bag assembly further comprises a second securing means connected to the support member for releasably securing the separation bag thereto.
- The first securing means and the second securing means are so arranged with respect to each other that the product tube forms a loop large enough for preventing the kinking thereof when the at least one product bag is secured to the support member by the first securing means and the separation bag is secured to the support member by the second securing means.
- The second securing means is adapted to secure the separation bag to the support member by a peripheral edge thereof.
- The separation bag is substantially annular or semi-annular, and the second securing means is adapted to secure the separation bag to the support member by an inner peripheral edge thereof.
- The second securing means is adapted to secure the separation bag to the support member by at least two areas thereof.
- The second securing means comprises two spaced apart protruding elements for engaging two apertures in a peripheral area of the separation bag.
- The bag assembly further comprises a handle connected to the support member and the two protruding elements extend from the handle.
- The first and the second securing means comprise the same two spaced apart protruding elements for engaging two apertures in a peripheral area of the separation bag and two holes in a peripheral area of the at least one product bag.
- The bag assembly further comprises a collection bag connected to the separation bag by a transfer tube.
- The bag-assembling device further comprises a third securing means connected to the support member for releasably securing the collection bag to the support member.
- The support member comprises an elongated body having a first and a second opposite lateral sides, the first securing means comprises two spaced apart protruding elements for engaging two holes in a peripheral area of the at least one product bag, and the third securing means comprises two spaced apart protruding elements for engaging two holes in a peripheral area of the collection bag, whereby the protruding elements of the first securing means extend from the first side of the elongated body and the protruding elements of the second securing means extend from the second side of the elongated body.
- The bag-assembling device further comprises a second securing means connected to the support member for releasably securing the separation bag to the support member and a third securing means connected to the support member for releasably securing the collection bag to the support member, whereby the second securing means and the third securing means are so arranged with respect to each other that the transfer tube forms a loop large enough for preventing the kinking thereof when the separation bag is secured to the support member by the second securing means and the collection bag is secured to the support member by the third securing means.
- The bag assembly further comprises a filter connected to a tube connected to the at least one product bag.
- The bag-assembling device further comprises a holder connected to the support member for releasably holding the filter.
- The mounting means and the filter holder are so arranged with respect to each other that the holder is located within the central cavity of a rotor when the bag-assembling device in mounted therein.
- The filter has a disk-like housing and the filter holder comprises a cylindrical portion for containing the filter so that the filter and the cylindrical portion of the holder are substantially concentric.
- The filter holder is connected to the support member so that a central axis of the cylindrical portion of the holder is substantially perpendicular to a rotation axis of the rotor when the bag-assembling device is mounted in a rotor.
- The filter holder and the first securing means are so arranged with respect to each other that the at least one product bag is substantially parallel to one of the circular side of the disk-like housing of the filter.
- The bag-assembling device further comprises a first winding support for coiling a portion of a tube connected to the filter.
- The bag-assembling device further comprises a second winding support for coiling a collection tube.
- The bag assembly further comprises a clamp for selectively closing the product tube connecting the at least one product bag to the separation bag.

Other features and advantages of the invention will appear from the following description and accompanying drawings, which are to be considered exemplary only.

In the accompanying drawings:
Figure 1 is a schematic view of a first set of bags for a bag assembly according to the invention;
Figure 2 is a schematic view of a second set of bags for a bag assembly according to the invention;
Figure 3 is a perspective view of the upper part of the rotor of a centrifuge adapted to the bag assembly according to the invention;
Figure 4 is a perspective view (side A) of a first bag-assembling device according to the invention;
Figure 5 is another perspective view (side B) of the first bag-assembling device according to the invention;
Figure 6 is a perspective view of a bag assembly comprising the first bag-assembling device, at an earlier stage of its manufacture;
Figure 7 is a perspective view of the bag assembly of figure 6, at a later stage of its manufacture;
Figure 8 is a perspective view of a second bag-assembling device according to the invention;
Figure 9 is a perspective view of a bag assembly comprising the second bag-assembling device, at an earlier stage of its manufacture;
Figure 10 is a perspective view of the bag assembly of figure 9, at a later stage of its manufacture;
Figure 11 is a perspective view of a third bag-assembling device according to the invention;
Figure 12 is a perspective view of a bag assembly comprising the third bag-assembling device and four bags connected thereto;
Figure 13 is a schematic perspective view of a fourth bag-assembling device according to the invention;
Figure 14 is a schematic top view of a bag assembly comprising the fourth bag-assembling device and three bags connected thereto;
Figure 15 is a perspective view (side A) of a fifth bag-assembling device according to the invention; and
Figure 16 is another perspective view (side B) of the fifth bag-assembling device according to the invention.

For the sake of clarity, the invention will be described with respect to a specific use, namely the separation of whole blood into a first component comprising plasma, a second component comprising platelets and a third component comprising red blood cell. It should be understood however that this specific use is exemplary only.

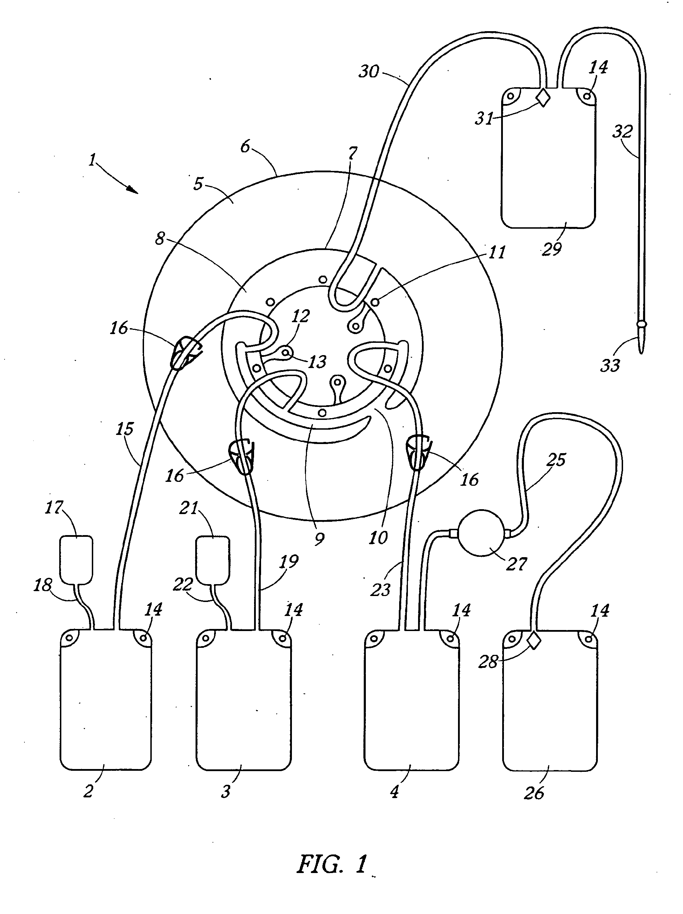

Figure 1 shows a first example of a set of bags adapted to the separation of whole blood into a plasma product, a platelet product and red blood cell product. This bag set comprises a separation bag 1 and three product bags 2, 3, 4 connected thereto. The separation bag 1 comprises a substantially annular separation chamber 5 having an outer circular edge 6 and an inner circular edge 7, and a disk-shaped connecting element 8 that is connected to the inner edge 7 of the annular chamber 5. The outer circular edge 6 and the inner circular edge 7 of the separation chamber 5 are substantially concentric. The disk-shaped connecting element 8 comprises a substantially semi-circular distribution channel 9 embedded therein, which communicates through a passage 10 with the annular chamber 5. The disk-shaped connecting element 8 further comprises a series of holes 11 for connecting the separation bag 1 to the rotor of a centrifuge. Three peripheral tongues 12 having an aperture 13 therethrough are attached to the inner periphery of the disk-shaped connecting element 8 for fastening the separation bag 1 to a bag-assembling device that will be later described.

Variants of the separation bag 1 may include:
- a separation chamber 5 having an outer circular edge 6 and an inner circular edge 7 that are eccentric;
- a separation chamber 5 having one or two radial walls extending from the inner edge 7 to the outer edge 6 so that the chamber, instead of being annular, has a C-shape with the C being more or less open.

Also the separation bag 1 can be shaped so as to fit either on a flat support surface or on a frusto-conical support surface of the rotor of a centrifuge.

The first product bag 2 is intended for containing the platelet product. It is flat and substantially rectangular and comprises a hole 14 at each of its upper corners for fastening the product bag 2 to a bag-assembling device (described hereunder). It is connected by a first product tube 15 to the separation bag 1. The first product tube 15, which is fitted with a clamp 16, has a first end connected to the upper edge of the first product bag 2 and a second end connected to a first end of the distribution channel 9. A first bag 17 for purging air is connected by a tube 18 to the upper edge of the first product bag 2.

The second product bag 3 is intended for containing the plasma product. It is flat and substantially rectangular and comprises a hole 14 at each of its upper corners for fastening the product bag 3 to a bag-assembling device (described hereunder). It is connected by a second product tube 19 to the separation bag 1. The second product tube 19, which is fitted with a clamp 16, has a first end connected to the upper edge of the second product bag 3 and a second end connected to the distribution channel 9, between the end of the first product tube 15 connected thereto and the passage 10 between the distribution channel 9 and the annular chamber 5. A second bag 21 for purging air is connected by a tube 22 to the upper edge of the second product bag 3.

The third product bag 4 is intended for containing the red blood cell product. It is flat and substantially rectangular and comprises a hole 14 at each of its upper corners for fastening the product bag 4 to a bag-assembling device (described hereunder). It is connected by a third product tube 23 to the separation bag 1. The third product tube 23, which is fitted with a clamp 16, has a first end connected to the upper edge of the third product bag 4 and a second end connected to a second end of the distribution channel 9.

The third product bag 4 is connected by a transfer tube 25 to an auxiliary product bag 26, which is flat, substantially rectangular and comprises a hole 14 at each of its upper corners for fastening the auxiliary product bag 26 to a bag-assembling device (described hereunder). The transfer tube 25 has a first end connected to the upper edge of the third product bag 4 and a second end connected to the upper edge of the auxiliary product bag 26. It has two segments respectively connected to the inlet and the outlet of a leukoreduction filter 27 (a filter for removing white blood cells). The auxiliary bag 26 contains a volume of storage solution for red blood cells. A plug 28 removable from within the auxiliary product bag 26 (so-called "frangible pin", for example) blocks a liquid flow through the transfer tube 25 and prevents the storage solution from flowing from the auxiliary product bag 26 into the third product bag 4. Alternatively, the auxiliary bag 26 does not contain a storage solution, and is fitted with a port connected to a sterile filter, through which a storage solution can be injected within the auxiliary bag 26 just before the red blood cells are transferred from the third product bag into the auxiliary bag 26.

The bag set further comprises a collection bag 29, which is intended for receiving a volume of whole blood from a donor (usually about 450 ml). The collection bag 29 is flat, substantially rectangular and comprises a hole 14 at each of its upper corners for fastening the collection bag 29 to a bag-assembling device (described hereunder). The collection bag 29 is connected to the separation bag 1 by a transfer tube 30 having one end attached to the upper edge of the collection bag 29 and the other end attached at the inner edge 7 of the annular chamber 5. The collection bag 29 contains a volume of anti-coagulant solution (typically about 70 ml of a solution of citrate phosphate dextrose for a blood donation of about 450 ml). A plug 31 removable from within the collection bag 29 (so-called "frangible pin", for example) blocks a liquid flow through the tube 30 and prevents the anti-coagulant solution from flowing from the collection bag 29 into the separation bag 1.

The bag set further comprises a collection tube 32 that is connected at one end to the upper edge of the collection bag 29 and comprises, at the other end, a needle protected by a sheath 33.

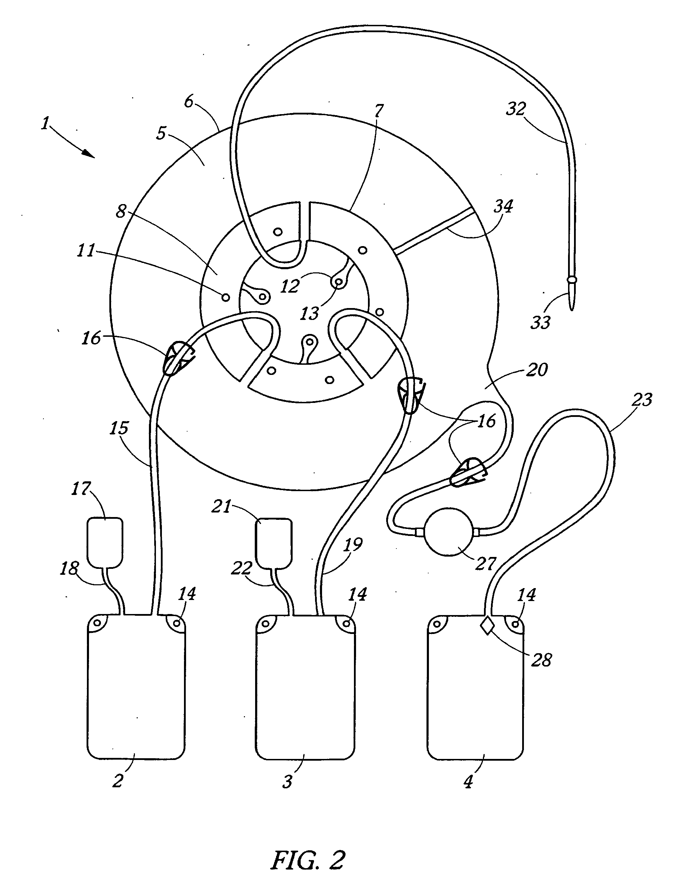

Figure 2 shows a second example of a set of bags adapted to the separation of whole blood into a plasma product, a platelet product and red blood cell product. This bag set comprises a separation bag 1 and three product bags 2, 3, 4 connected thereto. The separation bag 1 comprises a partially annular separation chamber 5 having a substantially circular outer edge 6, an inner circular edge 7 and a radial wall 34 extending from the inner edge 7 to the outer edge 6 so that the chamber 5 has a shape of a broken ring. The separation chamber 5 also comprises a funnel like extension 20 protruding outwardly from its outer edge 6 for helping evacuate a content of the separation chamber into a third product bag 4 to be described hereunder. The separation bag 1 further comprises a disk-shaped connecting element 8 that is connected to the inner edge 7 of the annular chamber 5. The disk-shaped connecting element 8 comprises a series of holes 11 for connecting the separation bag 1 to the rotor of a centrifuge. Three peripheral tongues 12 having an aperture 13 therethrough are attached to the inner periphery of the disk-shaped connecting element 8 for fastening the separation bag 1 to a bag-assembling device (described hereunder).

The first product bag 2 is intended for containing the platelet product. It is flat and substantially rectangular and comprises a hole 14 at each of its upper corners for fastening the product bag 2 to a bag-assembling device (hereunder described). It is connected by a first product tube 15 to the separation bag 1. The first product tube 15, which is fitted with a clamp 16, has a first end connected to the upper edge of the first product bag 2 and a second end connected to the inner edge 7 of the annular chamber 5. A first bag 17 for purging air is connected by a tube 18 to the upper edge of the first product bag 2.

The second product bag 3 is intended for containing the plasma product. It is flat and substantially rectangular and comprises a hole 14 at each of its upper corners for fastening the product bag 3 to a bag-assembling device (described hereunder). It is connected by a second product tube 19 to the separation bag 1. The second product tube 19, which is fitted with a clamp 16, has a first end connected to the upper edge of the second product bag 3 and a second end connected to the inner edge 7 of the annular chamber 5. A second bag 21 for purging air is connected by a tube 22 to the upper edge of the second product bag 3.

The third product bag 4 is intended for containing the red blood cell product. It is flat and substantially rectangular and comprises a hole 14 at each of its upper corners for fastening the product bag 4 to a bag-assembling device (described hereunder). It is connected by a third product tube 23 to the separation bag 1. The third product tube 23, which is fitted with a clamp 16, has a first end connected to the upper edge of the third product bag 4 and a second end connected to the tip of the funnel like extension 20 of the separation chamber 5. It comprises two segments respectively connected to the inlet and the outlet of a leukoreduction filter 27 (a filter for removing white blood cells). The third product bag 4 contains a volume of storage solution for red blood cells. A plug 28 removable from within the third product bag 4 (so-called "frangible pin", for example) blocks a liquid flow through the product tube 23 and prevents the storage solution from flowing from the third product bag 4 into the separation bag 1.

The bag set further comprises a collection tube 32 that is connected at one end to the inner edge 7 of the separation bag 1 and comprises, at the other end, a needle protected by a sheath 33.

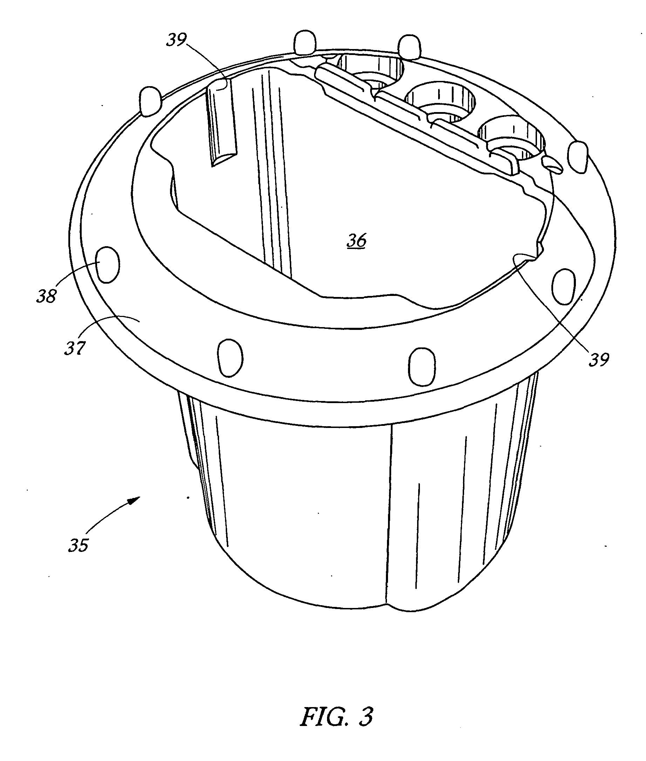

Figure 3 shows the central portion of the upper part of the rotor 35 of a centrifuge used for the separation a volume of blood into a plasma product, a platelet product and a red blood cell product. The central portion of this rotor 35 comprises a central container 36 intended for receiving the product bags 2, 3, 4, 26 and the filter 27 during the separation process. It further comprises an annular flange 37, connected to the upper rim of the container 36, for supporting the disk-shaped connecting element 8 of a separation bag 1. The annular flange 37 is fitted with a series of rounded pins 38 for engaging the holes 11 in the connecting element 8 of the separation bag 1 so as to prevent the separation bag 1 from moving with respect to the rotor during the centrifugation thereof. The rotor 35 also comprises a mounting means for cooperating with a complementary mounting means of a bag-assembling device of the invention, which will be described hereunder. The mounting means of the rotor 35 comprises two slots 39 that are recessed within the wall of the container 36. The slots 39 are substantially equidistant with respect to and in the same plane as the rotation axis of the rotor 35.

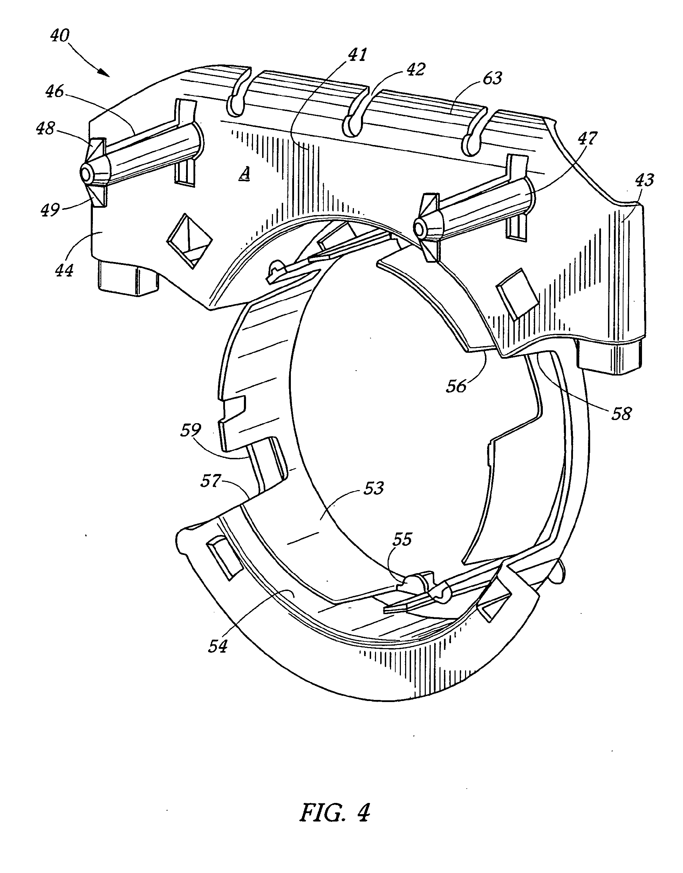

Figures 4 to 16 show five embodiments of a bag-assembling device according to the invention. The bag-assembling device has three main purposes: first, it is used, during the manufacture of the set of bags, for assembling in a determined order the product bags 2, 3, 4, 26 and the collection bag 29 to the separation bag 1; second, it is used, during the sterilization of the bags once connected together by the various tubes, for maintaining the bags in a fixed relationship with respect to each other so that the loops, formed in the tubes during the assembling stages, keep their curvature and the tubes do not run the risk of kinking; third, it is used, during the use of the bag assembly as a final product, for quickly loading and unloading the set of bags in the rotor 35 of a centrifuge, and for keeping the product bags 2, 3, 4, 26 in a determined position within the container 36 of the rotor 35 during the spinning thereof.

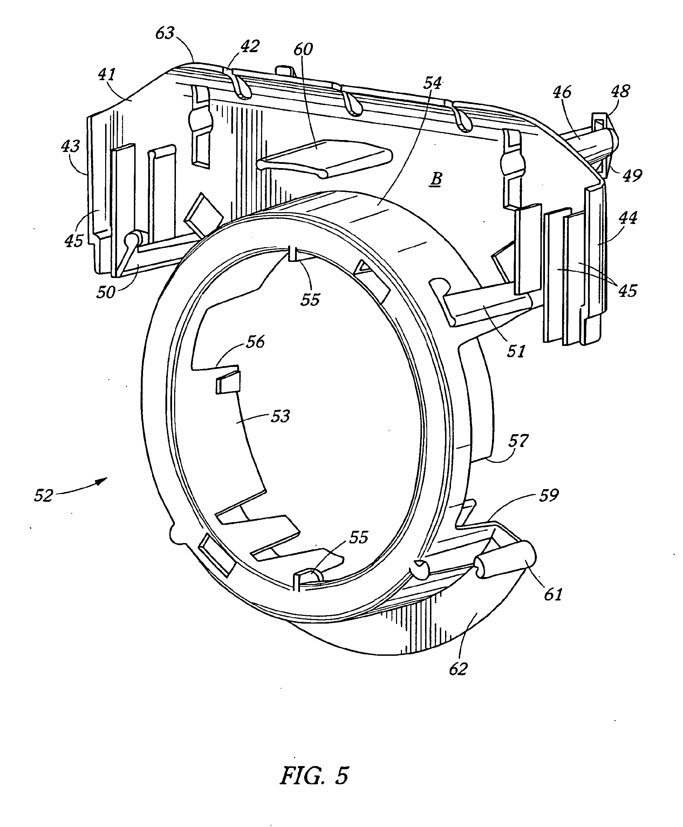

Figures 4 and 5 represent the two sides A and B of a first embodiment of a bag-assembling device 40 according to the invention. The bag-assembling device comprises a support member 41 having an elongated flat body connected to an upper ridge 63 that is bent. Three slits 42 are cut out in the bent ridge 63 for guiding the product tubes 15, 19, 23.

The bag-assembling device 40 further comprises a mounting means having two extensions 43, 44 connected to the lateral edges of the elongated flat body 41. The outer rims of the extensions 43, 44 are bent so as to be perpendicular to the elongated flat body 41. The extensions 43, 44 are fitted respectively with one or two gutter-like holders 45 parallel to their outer rims, in which a portion of tube or the needle sheath 33 can be snuggly engaged. The mounting means is for removably mounting the bag-assembling device 40 in the rotor 35 represented in figure 3. The shape of the extensions 43 and 44 is complementary of the shape of the slots 39 in the upper part of the wall of the container 36, so that they can be engaged thereto and form therewith a tongue-and-groove arrangement. When the bag-assembling device 40 is mounted in the rotor 35, the support member 41 is substantially located within the container 36, it extends across the aperture of the container 36, and perpendicularly intersects the rotation axis of the rotor 35.

The bag-assembling device 40 further comprises a first securing means connected to the support member 41 for releasably securing the product bags 2, 3, 4, 26 to the support member 41. The first securing means is comprised of two spaced apart protruding elements in the form of rods 46, 47 that perpendicularly extend from the side A of the elongated body 41, just beneath the bent upper ridge 63 thereof. The distance between the two rods 46, 47 is substantially the same as the distance between the holes 14 in the upper part of the product bags 2, 3, 4, 26. The cross-section of the rods 46, 47 substantially fits in the holes 14. The tip of each rod 46, 47 is fitted with an upper and a lower barbs 48, 49 for preventing a bag engaged on the rod from escaping therefrom during centrifugation of the bag assembly. The upper barb 48, which extends upwardly, is smaller than the lower barb 49, which extends downwardly, so as to facilitate the engagement of the rod 46, 47 in a hole 14 of a bag.

It results from the respective arrangement of the mounting means 43, 44 and the first securing means 46, 47 that the product bags 2, 3, 4, 26 occupy a determined position in the central cavity of a rotor when the assembling device and the bags secured thereto are mounted in the rotor. More specifically, when mounted in the rotor 35 represented in figure 3, the bags 2, 3, 4, 26 are very close to the rotation axis of the rotor so that the centrifugal forces exerted on the content of these bags during the separation process is minimal. This is of particular interest for a platelet product, which consists of platelets suspended in plasma, since the platelets tend to agglutinate when subjected to substantial centrifugation forces.

The bag-assembling device 40 further comprises a second securing means connected to the support member 41 for releasably securing a separation bag 1 thereto. In the embodiment of the bag-assembling device 40 represented in the figures 4, 5, 6, 7, the second securing means is also comprised of the two spaced apart rods 46, 47 that form the first securing means. Alternatively, the second securing means may be comprised of separate protruding elements. The use of the rods 46, 47 as a second securing means requires that the three tongues 12 extending from the inner periphery of the disk-shaped connecting element 8 of the bag 1 (in figures 1 and 2) are arranged as follows: first, the tongues 12 are spaced apart around the inner periphery of the disk-shaped connecting element 8 so that the separation bag 1 can be folded in two along a diameter and two of the tongues 12 are superposed; second, the distance between the holes 13 in the two superposed tongues 12 and the hole 13 in the third tongue 12 is substantially the same as the distance between the rods 46, 47.

The second securing means is useful during the sterilization and the subsequent manipulation and shipping of the bag assembly (i.e. the set of bags of the figure 1 or 2 secured to the bag-assembling device 40), since it keeps the separation bag 1 in a fixed position with respect to the product bags 2, 3, 4, 26 and allows for bending the various tubes interconnecting them in substantially stable loops so that the kinking of the tubes is prevented. Note that this result could also be achieved by placing the bag assembly, with only the product bags 2, 3, 4, 26 secured to the bag-assembling device 40, in a package so shaped as to keep the separation bag 1 in a fixed position with respect to the product bags 2, 3, 4, 26.

The bag-assembling device 40 further comprises a third securing means connected to the support member 41 for releasably securing the collection bag 25 thereto. The third securing means is comprised of two spaced apart protruding elements in the form of hooked pegs 50, 51 that perpendicularly extend from the side B of the elongated plate 41, just above the lower ridge thereof. The distance between the two pegs 50, 51 is substantially the same as the distance between the holes 14 in the upper part of the collection bag 29.

It results from the respective arrangement of the second securing means (rods 46, 47) and of the third securing means (pegs 50, 51) that the transfer tube 30 can be bent so as to form a loop large enough for preventing the kinking thereof when the separation bag 1 is secured to the support member 41 by the second securing means 46, 47 and the collection bag 29 is secured to the support member 41 by the third securing means (50, 51).

The bag-assembling device 40 further comprises a holder 52 connected to the support member 41 for releasably holding a filter 27 having a generally disk-shaped casing and diametrically opposed radial inlet and outlet. The holder 52 comprises a tubular section 53 having an internal diameter slightly larger than the diameter of the casing of the filter 27 and a width larger that the thickness of the filter 27. The tubular section 53 is connected to a concentric, larger tubular section 54 of the same width, which, in turn is connected to the lower edge of the elongated plate 41. The tubular sections 53, 54 are connected by one of their rims, their other rims are flush with the side A of the elongated body 41 and their central axis is perpendicular to the elongated body 41.

It results from the relative arrangement of the support member 41, the mounting means 43, 44 and the filter holder 52, that the holder 52 is located within the container 36 of the rotor 35, when the bag-assembling device 40 in mounted therein.

It results from the relative arrangement of the support member 41, the first securing means 46, 47, the third securing means 50, 51 and the filter holder 52, that the product bags 1, 2, 3, 26 and the collection bag 29 hang parallel to each other and to the circular sides of the filter 27, when the bag assembly is mounted within the rotor 35 of a centrifuge.

The tubular section 53 of the filter holder 52 is fitted with two stops 55 protruding inwardly so as to keep the filter 27 in a middle position within the tubular section 53. Since, as mentioned above, the thickness of the filter 27 is less that the width of the tubular section 53, the filter 27 does not contact the closest product bag and the collection bag; this disposition is of particular interest when the plastic material (e.g. PVC) of which the bags are made and the plastic material (e.g. polycarbonate) of which the casing of the filter 27 is made have a tendency to fuse together when in contact during a steam sterilization process.

The tubular section 53 of the filter holder 52 further comprises two diametrically opposed cut-outs 56, 57 for respectively allowing the two sections of the transfer tube 25 (product tube 23, in figure 2) connected to the inlet and to the outlet of the filter 27 to extend out of the filter holder 52. The larger tubular portion 54 also comprises two diametrically opposed cut-outs 58, 59 for the same purpose.

The bag-assembling device 40 further comprises a first winding support for coiling the downstream portion of the transfer tube 25 (product tube 23, in figure 2) connected to the outlet of the filter 27. The first winding support comprises the tubular gap defined between the outer surface of the smaller tubular section 53 and the inner surface of the larger tubular section 53.

The bag-assembling device further comprises a second winding support for coiling the collection tube 32. The second winding support comprises the larger tubular section 53 and several protruding guides arranged around its outer surface, namely: the two pegs 50, 51 of the third securing means, a tongue 60 perpendicularly protruding from the middle of the side B of the support member 41, and a pin 61 adjacent the cut-out 59, which is connected to a flange 62 extending downward from the rim of the larger tubular section 53 that is flush with the side A of the elongate body 41.

The bag-assembling device 40 described above can be manufactured in one piece by injection molding of a plastic material like polypropylene.

A bag assembly comprising the bag set represented in figure 1 and the bag-assembling device 40 is manufactured as follows.
- The disk-shaped filter 27 to which the two sections of the transfer tube 25 have been previously connected is inserted in the filter holder 52.
- The downstream section of the transfer tube 25, which is connected to the outlet of the filter 27, is coiled within the first winding support (annular gap between the tubular sections 53 and 54).
- The platelet product bag 2, to which the first air-purging bag 17 has been previously connected, is engaged on the two rods 46, 47 of the first securing means.
- The plasma product bag 3, to which the second air-purging bag 21 has been previously connected, is engaged on the two rods 46, 47 of the first securing means so as to stack against the platelet product bag 2. The first air purging bag 17 is inserted between the platelet product bag 2 and the plasma product bag 3 so that the tube 18 forms a large loop.
- The primary red blood cell product bag 4 is engaged on the two rods 46, 47 of the first securing means so as to stack against the plasma product bag 3. The second air purging bag 21 is inserted between the plasma product bag 2 and the primary red blood cell product bag 4 so that the tube 22 forms a large loop.
- The auxiliary red blood cell product bag 26 is engaged on the two rods 46, 47 of the first securing means so as to stack against the primary red blood cell product bag 4.
- The separation bag 1, to which the product tubes 15, 19, 23 and the transfer tube 30 have been previously connected, is engaged on the two rods 46, 47 of the second securing means so as to stack against the auxiliary red blood cell product bag 26.
- The free end of each product tube 15, 19, 23 is bonded to the upper edge of the corresponding product bag 2, 3, 4. The free ends of the upstream and downstream sections of the transfer tube 25 are respectively bonded to the upper edge of the primary red blood cell product bag 4 and of the auxiliary red blood cell product bag 26. As a result of the relative fixed positions of the filter 27, the product bags 2, 3, 4, 26, and the separation bag 1, the various tubes connecting them can be arranged so as to form large loops unlikely to kink during the sterilization of the bag assembly and the subsequent manipulations and shipping.
- The collection bag 29 is engaged on the two pegs 50, 51 of the third securing means.
- The collection tube 32 is coiled around the second winding support 53 and its free end is bonded to the upper edge of the collection bag 29. The free end of the transfer tube 30 is bonded to the upper edge of the collection bag 29. Here also, as a result of the relative fixed positions of the collection bag 29 and the separation bag 1, the collection tube 32 and the transfer tube 30 can be arranged so as to form large loops unlikely to kink during the sterilization of the bag assembly and subsequent manipulations and shipping.
- A volume of anticoagulant solution is injected into the collection bag 29 and a volume of storage solution is injected into the auxiliary red blood cell bag 26.
- The bag assembly may be inserted in a first package and steam sterilized.
- After sterilization the (packaged) bag assembly is enclosed in a (second) gastight package so as to prevent the evaporation of the anticoagulant solution from the collection bag 29 and of the storage solution from the auxiliary bag 26.

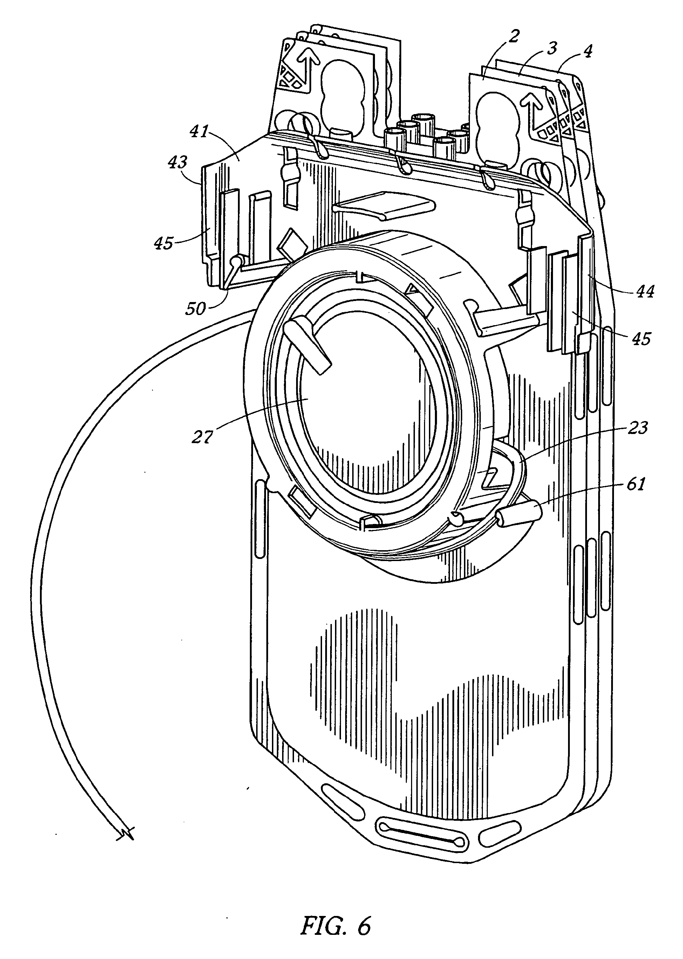

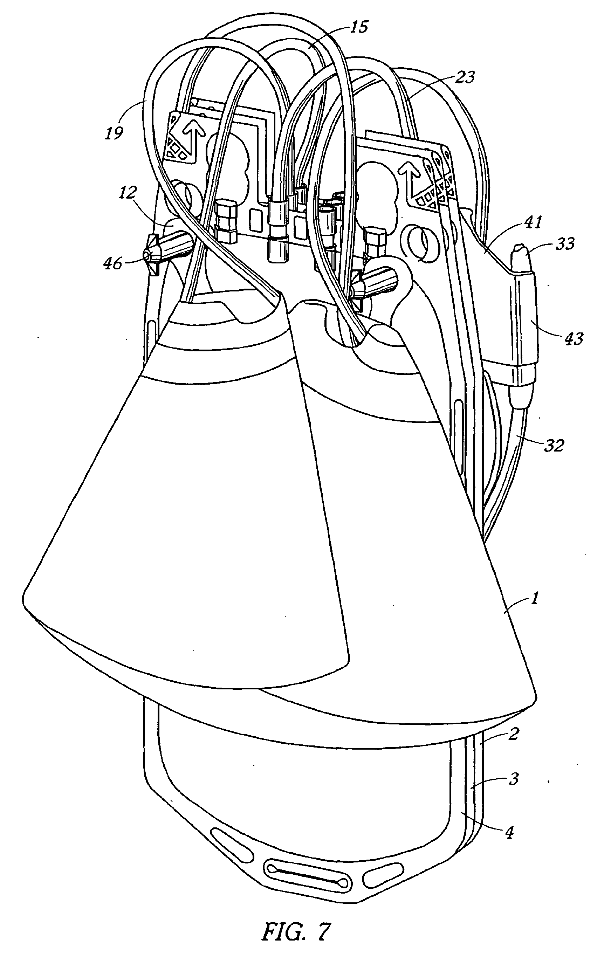

Figure 6 and 7 show two stages of the manufacture of a bag assembly comprising the bag-assembling device of figures 4 and 5 and the bag set of figure 2.

In figure 6, the disk-shaped filter 27 has been inserted in the filter holder 52 and the downstream section of the product tube 23, which is connected to the outlet of the filter 23, has been coiled within the first winding support (annular gap between the tubular sections 53 and 54); also the three product bags 2, 3, 4 have been engaged on the two rods 46, 47 of the first securing means.

In figure 7, the separation bag 1 has been engaged on the two rods 46, 47 so as to stack against the product bags 2, 3, 4; the free end of each product tube 15 and 19 has been bonded to the upper edge of the corresponding product bag 2 and 3; the ends of the product tube 23 have been respectively bonded to the upper edge of the third product bag 4 and to the funnel like extension 20 of the separation bag 1; finally, the collection tube 32 has been coiled around the second winding support 54 and the needle within the needle sheath 33 at the end of the collection tube 32 has been engaged in the needle holder 45.

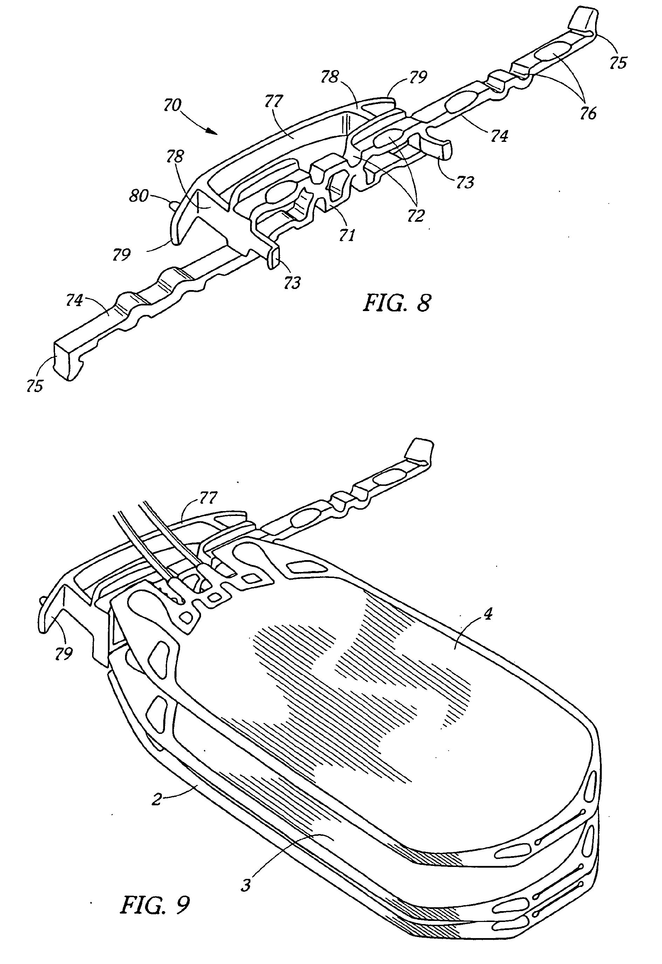

Figures 8, 9 and 10 show a second embodiment of a bag-assembling device 70 according to the invention. The bag-assembling device 70 comprises an elongated support member 71 having two lateral sides that are identical. Each side is generally flat except for an upper rim and a series of recesses 72 corresponding to the two tubes and the two sampling ports connected to the upper edge of a product bag.

The bag-assembling device 70 further comprises a mounting means for removably mounting the bag-assembling device to the rotor of a centrifuge. The mounting means is comprised of two resilient snap lock legs 73 that protrude downwards at each end of the support member 71.

The bag-assembling device 70 further comprises a first securing means for releasably securing to the support member 71 at least one product bag. The first securing means is comprised of two jaws 74 that are hinged at one end of each lateral side of the support member 71 and are fitted at their other end with a snap lock grip 75 by which they can be releasably fastened in a closed position to the support member 71. Each jaw 74 comprises recesses 76 that are symmetrical to the recesses 72 in the lateral sides of the support member 71. Each jaw 74 can secure one or two product bags to the support member 71.

The bag-assembling device 70 further comprises a handle 77 that extends above the support member 71 and is fastened thereto by two studs 78 that are aligned with the legs 73 of the mounting means. The purpose of this handle 77 is to facilitate the mounting and the withdrawal of a bag assembly in and from the rotor of a centrifuge.

The bag-assembling device 70 further comprises a second securing means connected to the support member 71 for releasably securing the separation bag 1 thereto. The second securing means comprises two lateral arms 79 extending from the ends of the handle 77 and two pins 80 protruding upwards from the lateral arms 79. The size and the distance between the two pins 80 correspond to a couple of holes 11 in the disk-shaped connecting element 8 of a separation bag 1.

One of the jaws 74 can be used to secure a collection bag 29 to the support member 71 and perform the function of the third securing means described above in reference to the figures 4 to 7.

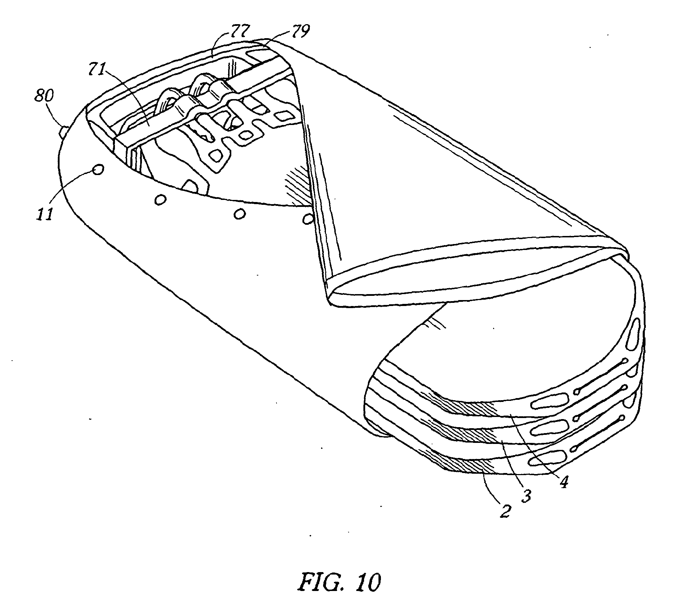

Figure 9 and 10 show a bag assembly including the bag-assembling device 70 and the bag set of figure 2 at two stages of its manufacture. In figure 9, two product bags 2, 3 have been secured to the support member 71 by one of the jaws 74, and a third product bag 4 is in place for being secured in turn to the support member 71 by the other jaw 74. In figure 10, the product bags 2, 3, 4 are connected to the separation bag 1, which is secured to the support member 71 by the two pins 80 and wrapped around the products bags. In this embodiment, the filter 27 is not secured to the support member 71 and is simply inserted between the product bags 2, 3, 4.

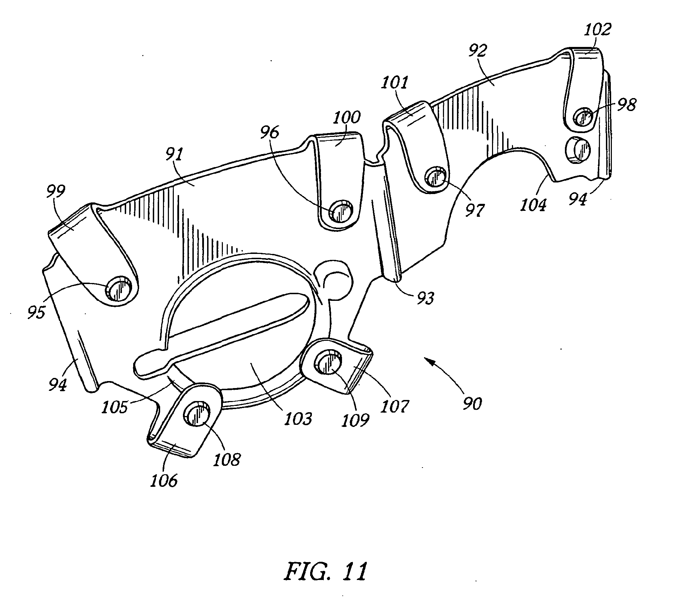

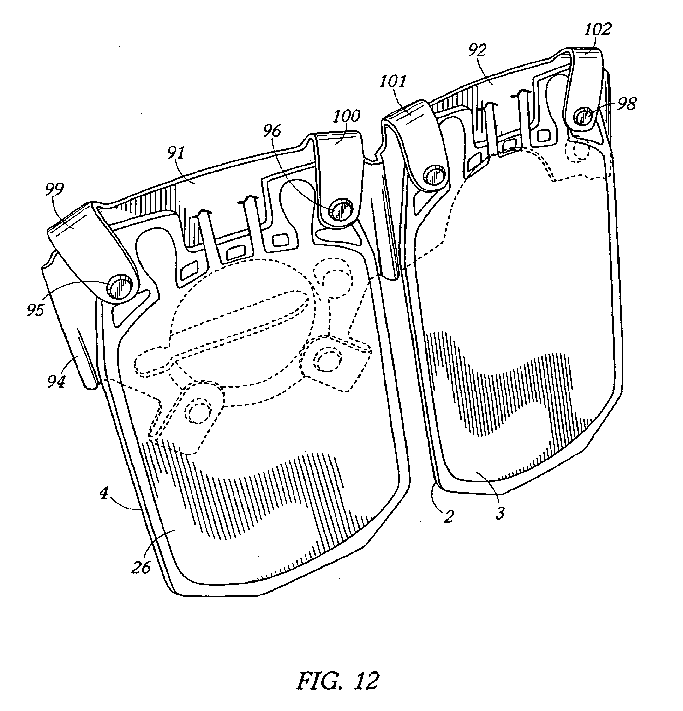

Figures 11 and 12 show a third embodiment of a bag-assembling device 90 according to the invention. The bag-assembling device 90 comprises a support member including two substantially flat flaps 91, 92 connected together by a hinge 93 that laterally protrudes.

Each flap comprises a lateral edge 94 parallel to the hinge 93, which is so designed that, when the two flaps 91, 92 are pressed against each other, the two lateral edges 94 have substantially the same shape as the hinge 93. In this embodiment of the invention, the mounting means by which the bag-assembling device 90 can be mounted in the rotor of a centrifuge is comprised of the hinge 93 and the two lateral edges 94 pressed against each other.

The bag-assembling device 90 further comprises a first securing means connected to the support member 91, 92 for releasably securing at least one product bag to the support member. The first securing means comprises two couples of spaced apart knobs 95, 96 and 97, 98 respectively connected to the outer surface of the flaps 91, 92, close to the upper edge thereof. The distance between the knobs 95, 96 and 97, 98 on each flap 91, 92 substantially corresponds to the distance between the two holes 14 at the upper edge of a product bag 2, 3, 4, 26. The first securing means also comprises four flexible straps 99, 100, 101, 102 corresponding to the knobs 95, 96, 97, 98. Each strap has one end connected to the upper edge of one of the flaps 91, 92 and another end fitted with a hole through which the corresponding knob can be forcibly engaged so as to prevent a bag engaged on the knob from escaping therefrom.

The bag-assembling device 90 further comprises a disk-shaped filter holder 103 that is integral with one of the flap 91 and recessed from the outer surface thereof. The other flap 92 comprises a cut-out 104 partially corresponding to the filter holder 103 so that, when the two flaps 91, 22 are pressed against each other their respective inner surfaces are in contact with each other.

The bag-assembling device 90 further comprises a winding support for coiling a section of the tube connected to a filter. The winding support comprises an annular rim 105 protruding from the outer surface of the flap 91 around the filter holder 103, and a means for releasably binding a coil of tube wound around the annular rim 105. The binding means comprises two flexible straps 106, 107 having one end connected to the lower edge of the flap 101 and having an other end fitted with a hole for forcibly engaging two knobs 108, 109 connected to the flap 91 close to the annular rim 105 of the winding support.

In addition to being used for securing products bags 2, 3, 4, 26 to the flaps 91, 92, each of the couple of knobs 95, 96 and 97, 98 can be used to secure a separation bag 1 and a collection bag 29 to the flaps 91, 92 and perform then the function of the second and third securing means described above in reference to the figures 4 to 7.

In figure 12, two products bags 2, 3 and 4, 26 are secured to each flap 91, 92 of the bag-assembling device 100.

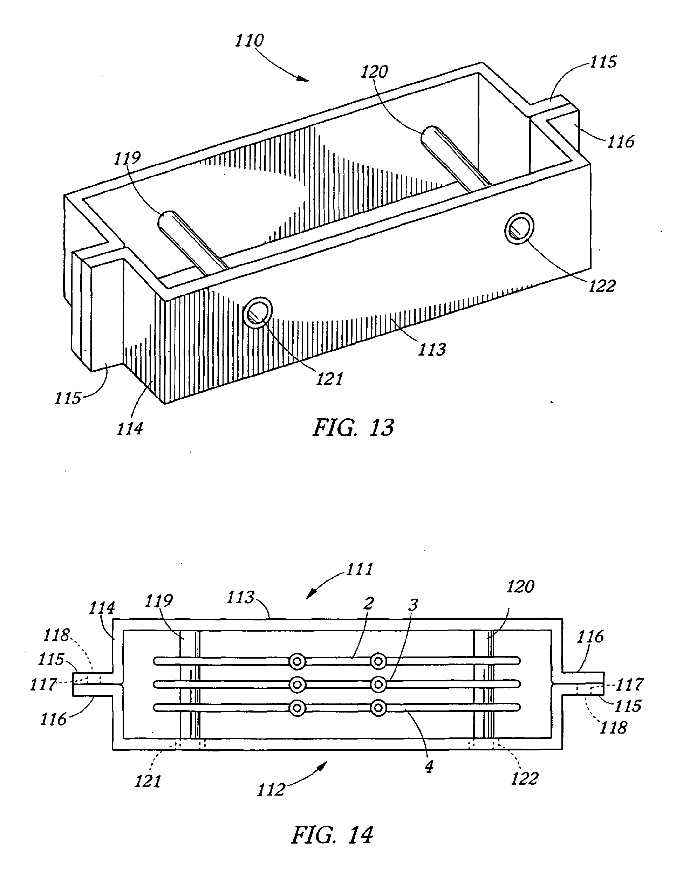

Figures 13 and 14 show a fourth embodiment of a bag-assembling device 110 according to the invention. The bag-assembling device 110 comprises a support member including two substantially identical U-shaped elements 111, 112 that are releasably connectable so as to define therebetween an oblong gap. Each U-shaped element comprises a straight flat base 113, two straight flat arms 114 perpendicularly connected to the base 113 at each end thereof of, and two connecting ears 115, 116 extending outwardly from the upper end of each arm 114. One of the connecting ears 115 comprises a hole 117 in the middle thereof and the other connecting ear 116 comprises a pin 118 in the middle thereof protruding outwardly and having a shape complementary to the shape of the hole 117 in the other connecting ear. The two identical U-shaped elements 111 and 112 of the support member can be connected by engaging the pin 118 of one U-shaped element 111, 112 in the hole 117 of the other U-shaped element 111, 112. The two ears 115, 116 connected to each other at both lateral ends of the support member form a mounting means by which the bag-assembly 110 can be removably mounted in a rotor of a centrifuge.

The bag assembling device 120 further comprises a bag-securing means in the form of two spaced apart rods 119, 120 perpendicularly connected to the base of one of the U-shaped elements 111 so as to extend across the gap within the U-shaped elements 111, 112. The distance between the rods 119, 120 is substantially equal to the distance between two securing holes 11, 13, 14 of any bag of a bag assembly. The rods 119, 120 are slightly longer than the width of the gap between two connected U-shaped elements 111, 112, and the U-shaped element 112 comprises two holes 121, 122 in its base 113 for receiving the tip of the rods 119, 120 protruding from the other U-shaped element 111.

In this embodiment of the bag-assembling device of the invention, the two rods 119, 120 can perform the function of the first, second and third securing means described above in reference to the figures 4 to 7.

The bag-assembling device 110 is adapted to hold stacked to each other all the bags of a bag set. In figure 14, the products bags 2, 3, 4 of the bag set of figure 2 are shown engaged on the rod 119, 120 of the bag-assembling device.

The bag-assembling device 110 does not comprise a holder for a filter 27, and the filter 27 simply hangs within the central cavity of the rotor when the bag assembly is mounted therein.

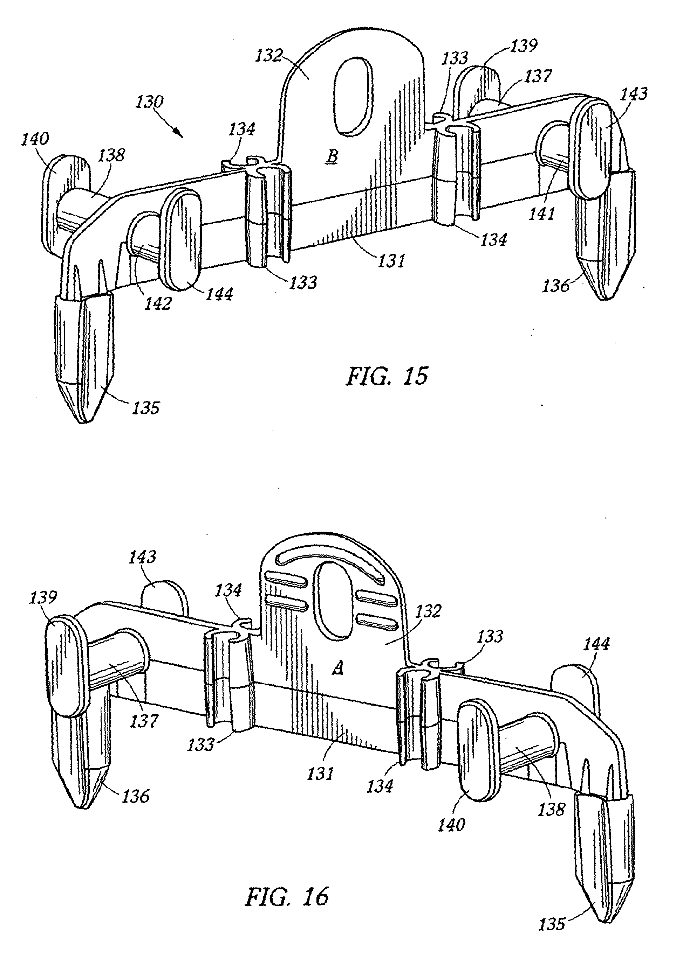

Figures 15 and 16 represent the two sides A and B of a fifth embodiment of a bag-assembling device 130 according to the invention. The bag-assembling device 130 comprises a support member having an elongated flat body 131. A flat U-shaped handling appendage 132 is connected to the elongated body 131 in the middle thereof so as to protrude upwardly when the bag-assembling device 130 is mounted in the rotor of a centrifuge. The elongated flat body 131 is fitted on both sides A and B with two parallel gutter-like holders 133, 134 that are perpendicular to a longitudinal axis of the elongated flat body 131 and extend in a central portion of the elongated flat body 131, substantially in alignment with the lateral edges of the U-shaped handling appendage 132, respectively. The gutter-like holders 133, 134 are so dimensioned that a portion of tube or the needle sheath 33 can be snuggly engaged therein.

The bag-assembling device 130 further comprises a mounting means for removably mounting the bag-assembling device 130 in the rotor of a centrifuge. The mounting means comprises two fang-like extensions 135, 136 connected to both ends of the elongated flat body 131 so as to protrude downwardly when the bag-assembling device 130 is mounted in the rotor of a centrifuge. This mounting means is used when the central container of the rotor of a centrifuge, like in figure 3, comprises two diametrally opposed slots or recesses for receiving the fang-like extensions 135, 136.

The bag-assembling device 130 further comprises a first securing means connected to the support member 131 for releasably securing the product bags 2, 3, 4, 26 to the support member 131. The first securing means is comprised of two spaced apart protruding elements in the form of rods 137, 138 that perpendicularly extend from the side A of the elongated body 131. The distance between the two rods 137, 138 is substantially the same as the distance between the holes 14 in the upper part of the product bags 2, 3, 4, 26. The cross-section of the rods 137, 138 substantially fits in the holes 14. The tip of each rod 137, 138 is fitted with a retaining element 139, 140 for preventing a bag engaged on the rod from escaping therefrom during centrifugation of the bag assembly. Each retaining element 139, 140 is comprised of a plate having rounded ends that is perpendicularly connected to the corresponding rod 137, 138 in such a way that the portion of the plate that extends upwardly is longer the portion of the plate that extends downwardly when the bag-assembling device 130 is mounted in the rotor of a centrifuge.

Alternatively, the first securing means 137, 138, 139, 140 also performs the function of the mounting means described above, when the central container of the rotor comprises a portion of wall that is substantially flat and has two slots or recesses so shaped and positioned as to receive and lock the tip of the rods 137, 138. When the tips of the rods 137, 138 are engaged in the two recesses, the support member 131 substantially extends in parallel to the flat wall of the central container of the rotor.

It results from the respective arrangement of the mounting means 135, 136 and the first securing means 137, 138, 139, 140 that the product bags 2, 3, 4, 26 occupy a determined position in the central cavity of a rotor when the assembling device and the bags secured thereto are mounted in the rotor.

The bag-assembling device 130 further comprises a second securing means connected to the support member 131 for releasably securing a separation bag 1 thereto. In the embodiment of the bag-assembling device 130 represented in the figures 15, 16, the second securing means is also comprised of the two spaced apart rods 137, 138 that form the first securing means. Alternatively, the second securing means may be comprised of separate protruding elements.

The bag-assembling device 130 further comprises a third securing means connected to the support member 131 for releasably securing the collection bag 25 thereto. The third securing means is comprised of two spaced apart protruding elements in the form of rods 141, 142 that perpendicularly extend from the side B of the elongated body 131 along the same axis as the rods 137, 138 of the first securing means, respectively. Like the rods 137, 138 of the first securing means, the tips of the rods 141, 142 are fitted with retaining elements 143, 144 for preventing a bag engaged on the rod from escaping therefrom during centrifugation of the bag assembly. Overall, the third securing means is identical to the first securing means save for the length of the rods, which is longer in the first securing means than in the third securing means.

The bag-assembling device 130 does not comprise a holder for a filter 27, and the filter 27 simply hangs within the central cavity of the rotor when the bag assembly is mounted therein.

It will be apparent to those skilled in the art that various modifications can be made to the bag-assembling device and method described herein. Thus, it should be understood that the invention is not limited to the subject matter discussed in the specification. Rather, the present invention is intended to cover modifications and variations within the scope of the claims.

## Claims

1. A bag assembly for the separation of a composite liquid into at least two components, said bag assembly comprising:
a set of bags comprising:
a separation bag (1);
at least one product bag (2, 3, 4, 26), and
a product tube (15, 19, 23, 25) connecting the separation bag (1) to the at least one product bag (2, 3, 4, 26);
the bag assembly **characterized by**:
a bag-assembling device (40; 70; 90; 110; 130) for assembling the set of bags, said bag-assembling device comprising:
a support member (41; 71; 91, 92; 111, 112; 131);
a mounting means (43, 44; 73; 93, 94; 115, 116; 135, 136), connected to the support member (41; 71; 91, 92; 111, 112; 131), for removably mounting the bag-assembling device (40; 70; 90; 110; 130) in a rotor of a centrifuge having a central cavity for containing the at least one product bag (2, 3, 4, 26); and
a first securing means (46, 47; 74; 95, 96, 97, 98; 119, 120; 137, 138) connected to the support member (41; 71; 91, 92; 111, 112; 131) for releasably securing the at least one product bag (2, 3, 4, 26) to the support member (41; 71; 91, 92; 111, 112; 131),
wherein the mounting means (43, 44; 73; 93, 94; 115, 116; 135, 136) and the first securing means (46, 47; 74; 95, 96, 97, 98; 119, 120; 137, 138) are connected to the support member so that the at least one product bag (2, 3, 4, 26) occupies a determined position in the central cavity of a rotor when the mounting means mounts the bag-assembling device (40; 70; 90; 110; 130) therein.

2. A bag assembly according to claim 1, comprising a plurality of product bags, wherein the first securing means (46, 47; 74; 95, 96, 97, 98; 119, 120; 137, 138) is adapted to releasably secure the product bags (2, 3, 4, 26) to the support member (41; 71; 91, 92; 111, 112; 131) so that they form a compact stack.

3. A bag assembly according to claim 1 or claim 2, wherein the first securing means (46, 47; 74; 95, 96, 97, 98; 119, 120; 137, 138) is adapted to secure at least one product bag (2, 3, 4, 26) to the support member (41; 71; 91, 92; 111, 112; 131) by a peripheral edge thereof.

4. A bag assembly according to any one of claims 1 to 3, wherein the at least one product bag (2, 3, 4, 26) is substantially rectangular and the first securing means (46, 47; 74; 95, 96, 97, 98; 119, 120; 137, 138) is adapted to secure the product bag (2, 3, 4, 26) to the support member (41; 71; 91, 92; 111, 112; 131) by an upper edge thereof.

5. A bag assembly according to any one of claims 1 to 4, wherein the first securing means (46, 47; 74; 95, 96, 97, 98; 119, 120; 137, 138) is adapted to secure at least one product bag (2, 3, 4, 26) to the support member (41; 71; 91, 92; 111, 112; 131) by at least two areas thereof.

6. A bag assembly according to claim 5, wherein the first securing means comprises at least two spaced apart protruding elements (46, 47; 95, 96, 97, 98; 119, 120; 137, 138) for engaging two holes (14) in a peripheral area of at least one product bag (2, 3, 4, 26).

7. A bag assembly according to claim 6, wherein the first securing means further comprises at least two straps (99, 100, 101, 102), each strap having a first end connected to the support member (111, 112) and a second end connectable to one of the protruding elements (95, 96, 97, 98) so as to prevent a bag engaged on the protruding element from escaping therefrom.

8. A bag assembly according to claim 6, wherein the support member comprises an elongated body (41; 131) having two opposite lateral sides (A, B), the first and second protruding elements comprise two rods (46, 47; 137, 138) extending substantially perpendicularly from one side (A) of the body (41; 131) of the support member, and each rod has a tip fitted with a retaining extension (48, 49; 139, 140) for preventing a bag engaged on the rods (46, 47; 137, 138) from escaping therefrom.

9. A bag assembly according to claim 6, wherein the support member comprises a first and a second U-shaped elements (111, 112) having a straight flat base (113), the first and a second U-shaped elements (111, 112) are releasably connectable so that their respective straight flat base (113) are substantially parallel and define an oblong gap therebetween, and the two spaced apart protruding elements comprise two substantially parallel rods (119, 120) that are connected to the straight flat base (113) of the first U-shaped elements (111) and extend across the oblong gap to the straight flat base (113) of the second U-shaped elements (112).

10. A bag assembly according to claim 6, wherein the support member comprises a first and second flaps (91, 92) hinged together and the at least two spaced apart protruding elements (95, 96, 97, 98) extend from an outside surface of the flaps (91, 92).

11. A bag assembly according to claim 10, comprising a plurality of product bags (2, 3, 4, 26), wherein the first securing means comprises four spaced apart protruding elements (95, 96, 97, 98), two protruding elements (95, 96) extending from the first flap (91) and two protruding elements (97, 98) extending from the second flap (92).

12. A bag assembly according to any one of claims 1 to 4, wherein the first securing means comprises at least one a jaw (74) for releasably grasping at least one product bag (2, 3, 4, 26) along a portion of a peripheral edge thereof.

13. A bag assembly according to claim 12, comprising a plurality of product bags (2, 3, 4, 26), wherein the support member (71) comprises an elongated body having two lateral sides and the first securing means comprises two jaws (74) respectively hinged to either side of the elongated body.

14. A bag assembly according to any one of claims 1 to 13, wherein the mounting means (43, 44; 73; 93, 94; 115, 116; 135, 136) is connected to the support member (41; 71; 91, 92; 111, 112; 131) so that the support member (41; 71; 91, 92; 111, 112; 131) is adapted to intersect a rotation axis of the rotor when the mounting means mounts the bag-assembling device (40; 70; 90; 110; 130) in the rotor.

15. A bag assembly according any one of claims 1 to 14, wherein:
the support member comprises an elongated portion (41; 71; 91, 92; 111, 112; 131) having two lateral ends, and
the mounting means comprises two extensions (43, 44; 73; 93, 94; 115, 116; 135, 136) extending at both ends of the elongated portion of the support member, and being adapted to engage two corresponding recesses in a wall of a central cavity of a rotor so that the support member (41; 71; 91, 92; 111, 112; 131) extends across the cavity of the rotor when the lateral extensions are engaged in the recesses thereof.

16. A bag assembly according to any one of claims 1 to 14, wherein:
the support member comprises an elongated body (131) having two opposite lateral sides (A, B),
the mounting means comprises a first and second protruding elements (137, 138) extending from one side (A) of the body of the support member (131), wherein the protruding elements (137, 138) have tips adapted to engage two recesses in a portion of a wall of a central cavity of a rotor so that the support member (131) extends substantially along the portion of wall when the protruding elements (137, 138) are engaged in the recesses thereof.

17. A bag assembly according to any one of claims 1 to 16, wherein the mounting means (43, 44; 73; 93, 94; 115, 116; 135, 136) and the first securing means (46, 47; 74; 95, 96, 97, 98; 119, 120; 137, 138) are connected to the support member so that the at least one product bag (2, 3, 4, 26) hangs substantially in an upright position when the mounting means mounts the bag-assembling device (40; 70; 90; 110; 130) in the rotor of a centrifuge.

18. A bag assembly according to any one of claims 1 to 17, further comprising a second securing means (79, 80) connected to the support member (41; 71; 91, 92; 111, 112; 131) for releasably securing the separation bag (1) thereto.

19. A bag assembly according to claim 18, wherein the first securing means (46, 47; 74; 95, 96, 97, 98; 119, 120; 137, 138) and the second securing means (79, 80) are connected to the support member so that the product tube (15, 19, 23, 25) forms a loop large enough for preventing the kinking thereof when the at least one product bag (2, 3, 4, 26) is secured to the support member (41; 71; 91, 92; 111, 112; 131) by the first securing means (46, 47; 74; 95, 96, 97, 98; 119, 120; 137, 138) and the separation bag (1) is secured to the support member (41; 71; 91, 92; 111, 112; 131) by the second securing means (79, 80).

20. A bag assembly according to claim 18 or claim 19, wherein the second securing means (79, 80) is adapted to secure the separation bag (1) to the support member (41; 71; 91, 92; 111, 112; 131) by a peripheral edge thereof.

21. A bag assembly according to any one of claims 18 to 20, wherein the separation bag (1) is substantially annular or semi-annular and the second securing means (79, 80) is adapted to secure the separation bag (1) to the support member (41; 71; 91, 92; 111, 112; 131) by an inner peripheral edge thereof.

22. A bag assembly according to any one of claims 18 to 21, wherein the second securing means (79, 80) is adapted to secure the separation bag (1) to the support member (41; 71; 91, 92; 111, 112; 131) by at least two areas thereof.

23. A bag assembly according to claim 22, wherein the second securing means comprises two spaced apart protruding elements (80) for engaging two apertures (11; 13) in a peripheral area of the separation bag (1).

24. A bag assembly according to claim 23, further comprising a handle (77) connected to the support member (71) and the two protruding elements (80) extend from the handle (77).

25. A bag assembly according to claim 1, wherein the first securing means comprises two spaced apart protruding elements (46, 47; 95, 96, 97, 98; 119, 120; 137, 138) for engaging two apertures (13) in a peripheral area of the separation bag (1) and two holes (14) in a peripheral area of the at least one product bag (2, 3, 4, 26).

26. A bag assembly according to any one of claims 1 to 25, further comprising a collection bag (29) connected to the separation bag (1) by a transfer tube (30).

27. A bag assembly according to claim 26, wherein the bag-assembling device (40; 70; 90; 110; 130) further comprises a third securing means (50, 51; 141, 142) connected to the support member (41; 71; 91, 92; 111, 112; 131) for releasably securing the collection bag (29) to the support member (41; 71; 91, 92; 111, 112; 131).

28. A bag assembly according to claim 27, wherein the support member comprises an elongated body (41, 131) having a first and a second opposite lateral sides (A, B), the first securing means comprises two spaced apart protruding elements (46, 47; 137, 138) for engaging two holes (14) in a peripheral area of the at least one product bag (2, 3, 4, 26), and the third securing means comprises two spaced apart protruding elements (50, 51; 141, 142) for engaging two holes (14) in a peripheral area of the collection bag (29), whereby the protruding elements (46, 47; 137, 138) of the first securing means extend from the first side (A) of the elongated body (41, 131) and the protruding elements (50, 51; 141, 142) of the third securing means extend from the second side (B) of the elongated body (41, 131).

29. A bag assembly according to any one of claims 1 to 17, wherein the first securing means releasably secures the separation bag (1) to the support member (41; 71; 91, 92; 111, 112; 131), and the bag assembly device further comprises a third securing means (50, 51; 141, 142) connected to the support member (41; 71; 91, 92; 111, 112; 131) for releasably securing the collection bag (29) to the support member (41; 71; 91, 92; 111, 112; 131), whereby the first securing means (46, 47; 79, 80; 95, 96, 97, 98; 119, 120; 137, 138) and the third securing means (50, 51; 74; 95, 96, 97, 98; 119, 120; 141, 142) are connected to the support member so that the transfer tube (30) forms a loop large enough for preventing the kinking thereof when the separation bag (1) is secured to the support member (41; 71; 91, 92; 111, 112; 131) by the first securing means (46, 47; 79, 80; 95, 96, 97, 98; 119, 120; 137, 138) and the collection bag (29) is secured to the support member (41; 71; 91, 92; 111, 112; 131) by the third securing means (50, 51; 74; 95, 96, 97, 98; 119, 120; 141, 142).

30. A bag assembly according to any one of claims 1 to 29, further comprising a filter (27) connected to a tube (23; 25) connected to the at least one product bag (4; 26).

31. A bag assembly according to claim 30, wherein the bag-assembling device (40; 90) further comprises a holder (52; 103) connected to the support member (41; 91) for releasably holding the filter (27).

32. A bag assembly according to claim 31, wherein the mounting means (43, 44; 93, 94) and the filter holder (52; 103) are connected to the support member so that the holder (52; 103) is located within the central cavity of a rotor when the bag-assembling device (40; 90) in mounted therein.

33. A bag assembly according to claim 32, wherein the filter (27) has a disk-like housing and the filter holder comprises a cylindrical portion (53, 105) for containing the filter (27) so that the filter (27) and the cylindrical portion (53, 105) of the holder are substantially concentric.

34. A bag assembly according to claim 33, wherein the filter holder is connected to the support member (41; 91) so that a central axis of the cylindrical portion (53, 103) of the holder is substantially perpendicular to a rotation axis of the rotor when the bag-assembling device (40; 90) is mounted in a rotor.

35. A bag assembly according to claim 33 or claim 34, wherein the filter holder (52, 103) and the first securing means (46, 47; 95, 96, 97, 98) are connected to the support member so that the at least one product bag (2, 3, 4, 26) is substantially parallel to one of the circular side of the disk-like housing of the filter (27).

36. A bag assembly according to any one of claims 30 to 35, wherein the bag-assembling device (40; 90) further comprises a first winding support (53, 54; 105) for coiling a portion of a tube (23; 25) connected to the filter.

37. A bag assembly according to any one of claims 1 to 36, wherein the bag-assembling device (40) further comprises a second winding support (50, 51, 54, 60, 61, 62) for coiling a collection tube (32).

38. A bag assembly according to any one of claims 1 to 37, further comprising a clamp (16) for selectively closing the product tube (15, 19, 23) connecting the at least one product bag (2, 3, 4) to the separation bag (1).

## Patentansprüche

1. Beutelanordnung für die Trennung einer zusammengesetzten Flüssigkeit in wenigstens zwei Komponenten, wobei die Beutelanordnung umfasst:
einen Satz Beutel, der umfasst:
einen Abtrennbeutel (1);
wenigstens einen Produktbeutel (2, 3, 4, 26) und
ein Produktrohr (15, 19, 23, 25), das den Abtrennbeutel (1) mit dem wenigstens einen Produktbeutel (2, 3, 4, 26) verbindet;
wobei die Beutelanordnung **gekennzeichnet ist durch**:
eine Beutelzusammenbauvorrichtung (40; 70; 90; 110; 130) zum Zusammenbauen des Satzes Beutel, wobei die Beutelzusammenbauvorrichtung umfasst:
ein Stützelement (41; 71; 91, 92; 111, 112; 131);
Befestigungsmittel (43, 44; 73; 93, 94; 115, 116; 135, 136), die mit dem Stützelement (41; 71; 91, 92; 111, 112; 131) verbunden sind, um die Beutelzusammenbauvorrichtung (40; 70; 90; 110; 130) abnehmbar in einem Rotor einer Zentrifuge anzubringen, die einen zentralen Hohlraum besitzt, um den wenigstens einen Produktbeutel (2, 3, 4, 26) aufzunehmen; und
erste Sicherungsmittel (46, 47; 74; 95, 96, 97, 98; 119, 120; 137, 138), die mit dem Stützelement (41; 71; 91, 92; 111, 112; 131) verbunden sind, um den wenigstens einen Produktbeutel (2, 3, 4, 26) abnehmbar an dem Stützelement (41; 71; 91, 92; 111, 112; 131) zu befestigen,
wobei die Befestigungsmittel (43, 44; 73; 93, 94; 115, 116; 135, 136) und die ersten Sicherungsmittel (46, 47; 74; 95, 96, 97, 98; 119, 120; 137, 138) mit dem Stützelement verbunden sind, so dass der wenigstens eine Produktbeutel (2, 3, 4, 26) eine vorgegebene Position in dem zentralen Hohlraum eines Rotors einnimmt, wenn die Befestigungsmittel die Beutelzusammenbauvorrichtung (40; 70; 90; 110; 130) darin anbringen.

2. Beutelanordnung nach Anspruch 1, die mehrere Produktbeutel umfasst, wobei die ersten Sicherungsmittel (46, 47; 74; 95, 96, 97, 98; 119, 120; 137, 138) beschaffen sind, um die Produktbeutel (2, 3, 4, 26) abnehmbar an dem Stützelement (41; 71; 91, 92; 111, 112; 131) zu befestigen, so dass sie einen kompakten Stapel bilden.

3. Beutelanordnung nach Anspruch 1 oder Anspruch 2, wobei die ersten Sicherungsmittel (46, 47; 74; 95, 96, 97, 98; 119, 120; 137, 138) beschaffen sind, um wenigstens einen Produktbeutel (2, 3, 4, 26) mit einem Umfangsrand von ihm an dem Stützelement (41; 71; 91, 92; 111, 112; 131) zu befestigen.

4. Beutelanordnung nach einem der Ansprüche 1 bis 3, wobei der wenigstens eine Produktbeutel (2, 3, 4, 26) im Wesentlichen rechteckig ist und die ersten Sicherungsmittel (46, 47; 74; 95, 96, 97, 98; 119, 120; 137, 138) beschaffen sind, um den Produktbeutel (2, 3, 4, 26) mit einer Oberkante von ihm an dem Stützelement (41; 71; 91, 92; 111, 112; 131) zu befestigen.

5. Beutelanordnung nach einem der Ansprüche 1 bis 4, wobei die ersten Sicherungsmittel (46, 47; 74; 95, 96, 97, 98; 119, 120; 137, 138) beschaffen sind, um wenigstens einen Produktbeutel (2, 3, 4, 26) mit wenigstens zwei Bereichen von ihm an dem Stützelement (41; 71; 91, 92; 111, 112; 131) zu befestigen.

6. Beutelanordnung nach Anspruch 5, wobei die ersten Sicherungsmittel wenigstens zwei beabstandete vorstehende Elemente (46, 47; 95, 96, 97, 98; 119, 120; 137, 138) für den Eingriff in zwei Löcher (14) in einem Umfangsbereich wenigstens eines Produktbeutels (2, 3, 4, 26) umfassen.

7. Beutelanordnung nach Anspruch 6, wobei die ersten Sicherungsmittel ferner wenigstens zwei Bänder (91, 100, 101, 102) umfassen, wobei jedes Band ein erstes Ende, das mit dem Stützelement (111, 112) verbunden ist, und ein zweites Ende, das mit einem der vorstehenden Elemente (95, 96, 97, 98) verbindbar ist, besitzt, um zu verhindern, dass sich ein sich mit dem vorstehenden Element in Eingriff befindlicher Beutel von ihm löst.

8. Beutelanordnung nach Anspruch 6, wobei das Stützelement einen länglichen Körper (41; 131) umfasst, der zwei gegenüberliegende laterale Seiten (A, B) besitzt, das erste und das zweite vorstehende Element zwei Stäbe (46, 47; 137, 138) umfassen, die im Wesentlichen senkrecht von einer Seite (A) des Körpers 41; 131) des Stützelements verlaufen, und jeder Stab eine Spitze besitzt, die in eine Halteerweiterung (48, 49; 139, 140) eingepasst ist, um zu verhindern, dass sich ein sich mit den Stäben (46, 47; 134, 138) in Eingriff befindlicher Beutel von diesen löst.

9. Beutelanordnung nach Anspruch 6, wobei das Stützelement ein erstes und ein zweites U-förmiges Element (111, 112) mit einer geraden flachen Basis (113) umfasst, wobei das erste und das zweite U-förmige Element (111, 112) abnehmbar verbindbar sind, so dass ihre jeweiligen geraden flachen Basen (113) im Wesentlichen parallel sind und eine längliche Lücke dazwischen definieren, und die zwei beabstandeten vorstehenden Elemente zwei im Wesentlichen parallele Stäbe (119, 120) umfassen, die mit der geraden flachen Basis (113) des ersten U-förmigen Elements (111) verbunden sind und sich über die längliche Lücke zur geraden flachen Basis (113) des zweiten U-förmigen Elements (112) erstrecken.

10. Beutelanordnung nach Anspruch 6, wobei das Stützelement eine erste und eine zweite Klappe (91, 92) umfasst, die aneinander angelenkt sind, und sich die wenigstens zwei beabstandeten vorstehenden Elemente (95, 96, 97, 98) von einer Außenfläche der Klappen (91, 92) erstrecken.

11. Beutelanordnung nach Anspruch 10, die mehrere Produktbeutel (2, 3, 4, 26) umfasst, wobei die ersten Sicherungsmittel vier beabstandete vorstehende Elemente (95, 96, 97, 98) umfassen, wobei sich zwei vorstehende Elemente (95, 96) von der ersten Klappe (91) erstrecken und sich zwei vorstehende Elemente (97, 98) von der zweiten Klappe (92) erstrecken.

12. Beutelanordnung nach einem der Ansprüche 1 bis 4, wobei die ersten Sicherungsmittel wenigstens eine Klaue (74) umfassen, um wenigstens einen Produktbeutel (2, 3, 4, 26) entlang einem Abschnitt eines Umfangsrands von ihm abnehmbar zu ergreifen.

13. Beutelanordnung nach Anspruch 12, die mehrere Produktbeutel (2, 3, 4, 26) umfasst, wobei das Stützelement (71) einen länglichen Körper umfasst, der zwei laterale Seiten besitzt, und die ersten Sicherungsmittel zwei Klauen (74) umfassen, von denen jede an einer Seite des länglichen Körpers angelenkt ist.

14. Beutelanordnung nach einem der Ansprüche 1 bis 13, wobei die Befestigungsmittel (43, 44; 73; 93, 94; 115, 116; 135, 136) mit dem Stützelement (41; 71; 91, 92; 111, 112; 131) verbunden sind, so dass das Stützelement (41; 71; 91, 92; 111, 112; 131) beschaffen ist, um eine Drehachse des Rotors zu schneiden, wenn die Befestigungsmittel die Beutelzusammenbauvorrichtung (40; 70; 90; 110; 130) im Rotor anbringen.

15. Beutelanordnung nach einem der Ansprüche 1 bis 14, wobei:
das Stützelement einen länglichen Abschnitt (41; 71; 91, 92; 111, 112; 131) mit zwei lateralen Enden umfasst, und
die Befestigungsmittel zwei Erweiterungen (43, 44; 73; 93, 94; 115, 116; 135, 136) umfassen, die sich an beiden Enden des länglichen Abschnitts des Stützelements erstrecken und beschaffen sind, um mit zwei entsprechenden Aussparungen in einer Wand eines zentralen Hohlraums eines Rotors in Eingriff zu gelangen, so dass sich das Stützelement (41; 71; 91, 92; 111, 112; 131) über den Hohlraum des Rotors erstreckt, wenn sich die lateralen Erweiterungen in dessen Aussparungen in Eingriff befinden.

16. Beutelanordnung nach einem der Ansprüche 1 bis 14, wobei:
das Stützelement einen länglichen Körper (131) mit zwei gegenüberliegenden lateralen Seiten (A, B) besitzt,
die Befestigungsmittel ein erstes und ein zweites vorstehendes Element (137, 138) umfassen, die sich von einer Seite (A) des Körpers des Stützelements (131) erstrecken, wobei die vorstehenden Elemente (137, 138) Spitzen besitzen, die beschaffen sind, um mit zwei Aussparungen in einem Abschnitt einer Wand eines zentralen Hohlraums eines Rotors in Eingriff zu gelangen, so dass sich das Stützelement (131) im Wesentlichen entlang dem Abschnitt der Wand erstreckt, wenn sich die vorstehenden Elemente (137, 138) in dessen Aussparungen in Eingriff befinden.

17. Beutelanordnung nach einem der Ansprüche 1 bis 16, wobei die Befestigungsmittel (43, 44; 73; 93, 94; 115, 116; 135, 136) und die ersten Sicherungsmittel (46, 47; 74; 95, 96, 97, 98; 119, 120; 137, 138) mit dem Stützelement verbunden sind, so dass der wenigstens eine Produktbeutel (2, 3, 4, 26) im Wesentlichen in einer aufrechten Position hängt, wenn die Befestigungsmittel die Beutelzusammenbauvorrichtung (40; 70; 90; 110; 130) im Rotor einer Zentrifuge anbringen.

18. Beutelanordnung nach einem der Ansprüche 1 bis 17, die ferner zweite Sicherungsmittel (79, 80) umfassen, die mit dem Stützelement (41; 71; 91, 92; 111, 112; 131) verbunden sind, um den Abtrennbeutel (1) abnehmbar daran zu befestigen.

19. Beutelanordnung nach Anspruch 18, wobei die ersten Sicherungsmittel (46, 47; 74; 95, 96, 97, 98; 119, 120; 137, 138) und die zweiten Sicherungsmittel (79, 80) mit dem Stützelement verbunden sind, so dass das Produktrohr (15, 19, 23, 25) eine Schleife bildet, die groß genug ist, um ihr Knicken zu verhindern, wenn der wenigstens eine Produktbeutel (2, 3, 4, 26) durch die ersten Sicherungsmittel (46, 47; 74; 95, 96, 97, 98; 119, 120; 137, 138) am Stützelement (41; 71; 91, 92; 111, 112; 131) befestigt ist und der Abtrennbeutel (1) durch die zweiten Sicherungsmittel (79, 80) am Stützelement (41; 71; 91, 92; 111, 112; 131) befestigt ist.

20. Beutelanordnung nach Anspruch 18 oder Anspruch 19, wobei die zweiten Sicherungsmittel (79, 80) beschaffen sind, um den Abtrennbeutel (1) mit einem Umfangsrand von ihm an dem Stützelement (41; 71; 91, 92; 111, 112; 131) zu befestigen.

21. Beutelanordnung nach einem der Ansprüche 18 bis 20, wobei der Abtrennbeutel (1) im Wesentlichen ringförmig oder halbringförmig ist und die zweiten Sicherungsmittel (79, 80) beschaffen sind, um den Abtrennbeutel (1) mit einem inneren Umfangsrand von ihm an dem Stützelement (41; 71; 91, 92; 111, 112; 131) zu befestigen.

22. Beutelanordnung nach einem der Ansprüche 18 bis 21, wobei die zweiten Sicherungsmittel (79, 80) beschaffen sind, um den Abtrennbeutel (1) mit wenigstens zwei Bereichen von ihm an dem Stützelement (41; 71; 91, 92; 111, 112; 131) zu befestigen.

23. Beutelanordnung nach Anspruch 22, wobei die zweiten Sicherungsmittel zwei beabstandete vorstehende Elemente (80) für den Eingriff in zwei Öffnungen (11; 13) in einem Umfangbereich des Abtrennbeutels (1) umfassen.

24. Beutelanordnung nach Anspruch 23, die ferner einen Griff (77) umfasst, der mit dem Stützelement (71) verbunden ist, wobei sich die zwei vorstehenden Elemente (80) von dem Griff (77) erstrecken.

25. Beutelanordnung nach Anspruch 1, wobei die ersten Sicherungsmittel zwei beabstandete vorstehende Elemente (46, 47; 95, 96, 97, 98; 119, 120; 137, 138) für den Eingriff in zwei Öffnungen (13) in einem Umfangsbereich des Abtrennbeutels (1) und in zwei Löcher (14) im Umfangsbereich des wenigstens einen Produktbeutels (2, 3, 4, 26) umfassen.

26. Beutelanordnung nach einem der Ansprüche 1 bis 25, die ferner einen Auffangbeutel (29) umfasst, der durch ein Übertragungsrohr mit dem Abtrennbeutel (1) verbunden ist.

27. Beutelanordnung nach Anspruch 26, wobei die Beutelzusammenbauvorrichtung (40; 70; 90; 110; 130) ferner dritte Sicherungsmittel (50, 51; 141, 142) umfasst, die mit dem Stützelement (41; 71; 91, 92; 111, 112; 131) verbunden sind, um den Auffangbeutel (29) abnehmbar an dem Stützelement (41; 71; 91, 92; 111, 112; 131) zu befestigen.

28. Beutelanordnung nach Anspruch 27, wobei das Stützelement einen länglichen Körper (41, 131) umfasst, der eine erste und eine zweite gegenüberliegende laterale Seite (A, B) besitzt, die ersten Sicherungsmittel zwei beabstandete vorstehende Elemente (46, 47; 137, 138) für den Eingriff in zwei Löcher (14) in einem Umfangsbereich des wenigstens einen Produktbeutels (2, 3, 4, 26) umfassen und die dritten Sicherungsmittel zwei beabstandete vorstehende Elemente (50, 51; 141, 142) für den Eingriff in zwei Löcher (14) in einem Umfangsbereich des Auffangbeutels (29) umfassen, wodurch sich die vorstehenden Elemente (46, 47; 137, 138) der ersten Sicherungsmittel von der ersten Seite (A) des länglichen Körpers (41, 131) erstrecken und sich die vorstehenden Elemente (50, 51; 141, 142) der dritten Sicherungsmittel von der zweiten Seite (B) des länglichen Körpers (41, 131) erstrecken.

29. Beutelanordnung nach einem der Ansprüche 1 bis 17, wobei die ersten Sicherungsmittel den Abtrennbeutel (1) am Stützelement (41; 71; 91, 92; 111, 112; 131) abnehmbar befestigen und die Beutelzusammenbauvorrichtung ferner dritte Sicherungsmittel (50, 51; 141, 142) umfasst, die mit dem Stützelement (41; 71; 91, 92; 111, 112; 131) verbunden sind, um den Auffangbeutel (29) abnehmbar an dem Stützelement (41; 71; 91, 92; 111, 112; 131) zu befestigen, wodurch die ersten Sicherungsmittel (46, 47; 79, 80; 95, 96, 97, 98; 119, 120; 137, 138) und die dritten Sicherungsmittel (50, 51; 74; 95, 96, 97, 98; 119, 120; 141, 142) mit dem Stützelement verbunden sind, so dass das Übertragungsrohr (30) eine Schleife bildet, die groß genug ist, um ihr Knicken zu verhindern, wenn der Abtrennbeutel (1) durch die ersten Sicherungsmittel (46, 47; 79, 80; 95, 96, 97, 98; 119, 120; 137, 138) am Stützelement (41; 71; 91, 92; 111, 112; 131) befestigt ist und der Auffangbeutel (29) durch die dritten Sicherungsmittel (50, 51; 74; 95, 96, 97, 98; 119, 120; 141, 142) am Stützelement (41; 71; 91, 92; 111, 112; 131) befestigt ist.

30. Beutelanordnung nach einem der Ansprüche 1 bis 29, die ferner ein Filter (27) umfasst, das mit einem Rohr (23; 25) verbunden ist, das mit dem wenigstens einen Produktbeutel (4; 26) verbunden ist.

31. Beutelanordnung nach Anspruch 30, wobei die Beutelzusammenbauvorrichtung (40; 90) ferner eine Halterung (52; 103) umfasst, die mit dem Stützelement (41; 91) verbunden ist, um das Filter (27) abnehmbar zu halten.

32. Beutelanordnung nach Anspruch 31, wobei die Befestigungsmittel (43, 44; 93, 94) und die Filterhalterung (52; 103) mit dem Stützelement verbunden sind, so dass sich die Halterung (52; 103) in dem zentralen Hohlraum eines Rotors befindet, wenn die Beutelzusammenbauvorrichtung (40; 90) darin angebracht ist.

33. Beutelanordnung nach Anspruch 32, wobei das Filter (27) ein scheibenähnliches Gehäuse besitzt und die Filterhalterung einen zylindrischen Abschnitt (53, 105) umfasst, um das Filter (27) zu enthalten, so dass das Filter (27) und der zylindrische Abschnitt (53, 105) der Halterung im Wesentlichen konzentrisch sind.

34. Beutelanordnung nach Anspruch 33, wobei die Filterhalterung mit dem Stützelement (41; 91) verbunden ist, so dass eine Mittenachse des zylindrischen Abschnitts (53, 103) der Halterung zu einer Rotationsachse des Rotors im Wesentlichen senkrecht ist, wenn die Beutelzusammenbauvorrichtung (40; 90) in einem Rotor angebracht ist.

35. Beutelanordnung nach Anspruch 33 oder Anspruch 34, wobei die Filterhalterung (52, 103) und die ersten Sicherungsmittel (46, 47; 95, 96, 97, 98) mit dem Stützelement verbunden sind, so dass der wenigstens eine Produktbeutel (2, 3, 4, 26) im Wesentlichen zu einer der kreisförmigen Seiten des scheibenähnlichen Gehäuses des Filters (27) parallel ist.

36. Beutelanordnung nach einem der Ansprüche 30 bis 35, wobei die Beutelzusammenbauvorrichtung (40; 90) ferner einen ersten Wickelträger (53, 54; 105) umfasst, um einen Abschnitt eines Rohrs (23; 25), das mit dem Filter verbunden ist, aufzuwickeln.

37. Beutelanordnung nach einem der Ansprüche 1 bis 36, wobei die Beutelzusammenbauvorrichtung (40) ferner einen zweiten Wickelträger (50, 51, 54, 60, 61, 62) zum Aufwickeln eines Auffangrohrs (32) umfasst.

38. Beutelanordnung nach einem der Ansprüche 1 bis 37, die ferner eine Klemme (16) umfasst, um das Produktrohr (15, 19, 23), das den wenigstens einen Produktbeutel (2, 3, 4) mit dem Abtrennbeutel (1) verbindet, wahlweise zu schließen.

## Revendications

1. Ensemble de poches pour la séparation d'un liquide composite en au moins deux composants, ledit ensemble de poches comportant :
un groupe de poches comportant :
une poche de séparation (1) ;
au moins une poche de produit (2, 3, 4, 26), et
un tuyau de produit (15, 19, 23, 25) reliant la poche de séparation (1) à au moins une poche de produit (2, 3, 4, 26) ;
l'ensemble de poches étant **caractérisé par** :
un dispositif d'assemblage de poches (40 ; 70 ; 90 ; 110 ; 130) pour assembler le groupe de poches, ledit dispositif d'assemblage de poches comportant :
un élément de support (41 ; 71 ; 91 ; 92 ; 111 ; 112 ; 131) ;
des moyens de montage (43, 44 ; 73 ; 93, 94 ; 115, 116 ; 135, 136), reliés à l'élément de support (41 ; 71 ; 91, 92 ; 111, 112 ; 131), pour monter de manière amovible le dispositif d'assemblage de poches (40 ; 70 ; 90 ; 110 ; 130) dans un rotor d'une centrifugeuse ayant une cavité centrale pour contenir la au moins une poche de produit (2, 3, 4, 26) ; et
des premiers moyens de fixation (46, 47 ; 74 ; 95, 96, 97, 98 ; 119, 120 ; 137, 138) reliés à l'élément de support (41 ; 71 ; 91 ; 92 ; 111, 112 ; 131) pour fixer de manière détachable la au moins une poche de produit (2, 3, 4, 26) à l'élément de support (41 ; 71 ; 91 ; 92 ; 111, 112 ; 131),
dans lequel les moyens de montage (43, 44 ; 73; 93, 94 ; 115, 116 ; 135, 136) et les premiers moyens de fixation (46, 47 ; 74 ; 95, 96, 97, 98 ; 119, 120 ; 137, 138) sont reliés à l'élément de support de sorte que la au moins une poche de produit (2, 3, 4, 26) occupe une position déterminée dans la cavité centrale d'un rotor lorsque les moyens de montage montent le dispositif d'assemblage de poches (40 ; 70 ; 90 ; 110 ; 130) dans celui-ci.

2. Ensemble de poches selon la revendication 1, comportant une pluralité de poches de produit, dans lequel les premiers moyens de fixation (46, 47 ; 74 ; 95, 96, 97, 98 ; 119, 120 ; 137, 138) sont adaptés pour fixer de manière détachable les poches de produit (2, 3, 4, 26) à l'élément de support (41 ; 71 ; 91 ; 92 ; 111, 112 ; 131) de sorte qu'elles forment une pile compacte.

3. Ensemble de poches selon la revendication 1 ou la revendication 2, dans lequel les premiers moyens de fixation (46, 47 ; 74 ; 95, 96, 97, 98 ; 119, 120 ; 137, 138) sont adaptés pour fixer au moins une poche de produit (2, 3, 4, 26) à l'élément de support (41 ; 71 ; 91 ; 92 ; 111, 112 ; 131) par un bord périphérique de celui-ci.

4. Ensemble de poches selon l'une quelconque des revendications 1 à 3, dans lequel la au moins une poche de produit (2, 3, 4, 26) est sensiblement rectangulaire et les premiers moyens de fixation (46, 47; 74 ; 95, 96, 97, 98 ; 119, 120 ; 137, 138) sont adaptés pour fixer la poche de produit (2, 3, 4, 26) à l'élément de support (41 ; 71 ; 91 ; 92 ; 111, 112 ; 131) par un bord supérieur de celui-ci.

5. Ensemble de poches selon l'une quelconque des revendications 1 à 4, dans lequel les premiers moyens de fixation (46, 47 ; 74 ; 95, 96, 97, 98 ; 119, 120 ; 137, 138) sont adaptés pour fixer au moins une poche de produit (2, 3, 4, 26) à l'élément de support (41 ; 71 ; 91 ; 92 ; 111, 112 ; 131) par au moins deux zones de celui-ci.

6. Ensemble de poches selon la revendication 5, dans lequel les premiers moyens de fixation comportent au moins deux éléments saillants espacés (46, 47 ; 95, 96, 97, 98 ; 119, 120 ; 137, 138) pour s'engager dans deux trous (14) dans une zone périphérique d'au moins une poche de produit (2, 3, 4, 26).

7. Ensemble de poches selon la revendication 6, dans lequel les premiers moyens de fixation comportent en outre au moins deux brides (99, 100, 101, 102), chaque bride ayant une première extrémité reliée à l'élément de support (111, 112) et une seconde extrémité pouvant être reliée à l'un des éléments saillants (95, 96, 97, 98) de manière à empêcher une poche engagée sur l'élément saillant de s'échapper de celui-ci.

8. Ensemble de poches selon la revendication 6, dans lequel l'élément de support comporte un corps allongé (41 ; 131) ayant deux côtés latéraux opposés (A, B), les premier et second éléments saillants comportent deux tiges (46, 47 ; 137, 138) s'étendant sensiblement perpendiculairement depuis un côté (A) du corps (41 ; 131) de l'élément de support, et chaque tige a une pointe munie d'un prolongement de retenue (48, 49 ; 139, 140) pour empêcher une poche engagée sur les tiges (46, 47 ; 137, 138) de s'échapper de celles-ci.

9. Ensemble de poches selon la revendication 6, dans lequel l'élément de support comporte un premier et un second élément en forme de U (111, 112) ayant une base plate droite (113), le premier et le second élément en forme de U (111, 112) pouvant être reliés de manière détachable à leur base plate droite respective (113) sont sensiblement parallèles et définissent un espace oblong entre eux, et les deux éléments saillants espacés comportent deux tiges sensiblement parallèles (119, 120) lesquelles sont reliées à la base plate droite (113) des premiers éléments en forme de U (113) et s'étendent à travers l'espace oblong jusqu'à la base plate droite (113) des seconds éléments en forme de U (112).

10. Ensemble de poches selon la revendication 6, dans lequel l'élément de support comporte un premier et un second volet (91, 92) articulés ensemble et les au moins deux éléments saillants espacés (95, 96, 97, 98) s'étendent depuis une surface extérieure des volets (91, 92).

11. Ensemble de poches selon la revendication 10, comportant une pluralité de poches de produit (2, 3, 4, 26), dans lequel les premiers moyens de fixation comportent quatre éléments saillants espacés (95, 96, 97, 98), deux éléments saillants (95, 96) s'étendant depuis le premier volet (91) et deux éléments saillants (97, 98) s'étendant depuis le second volet (92).

12. Ensemble de poches selon l'une quelconque des revendications 1 à 4, dans lequel les premiers moyens de fixation comportent au moins une mâchoire (74) pour saisir de manière détachable au moins une poche de produit (2, 3, 4, 26) le long d'une partie d'un bord périphérique de celle-ci.

13. Ensemble de poches selon la revendication 12, comportant une pluralité de poches de produit (2, 3, 4, 26), dans lequel l'élément de support (71) comporte un corps allongé ayant deux côtés latéraux et les premiers moyens de fixation comportent deux mâchoires (114) articulées respectivement à l'un ou l'autre côté du corps allongé.

14. Ensemble de poches selon l'une quelconque des revendications 1 à 13, dans lequel les moyens de montage (43, 44, 73 ; 93, 94 ; 115, 116 ; 135, 136), sont reliés à l'élément de support (41 ; 71 ; 91, 92 ; 111, 112 ; 131) de sorte que l'élément de support (41 ; 71 ; 91, 92 ; 111, 112 ; 131) est adapté pour intersecter un axe de rotation du rotor lorsque les moyens de montage montent le dispositif d'assemblage de poches (40 ; 70 ; 90 ; 110 ; 130) dans le rotor.

15. Ensemble de poches selon l'une quelconque des revendications 1 à 14, dans lequel :
l'élément de support comporte une partie allongée (41 ; 71 ; 91 ; 92 ; 111, 112 ; 131) ayant deux extrémités latérales, et
les moyens de montage comportent deux prolongements (43, 44, 73 ; 93, 94 ; 115, 116 ; 135, 136) s'étendant aux deux extrémités de la partie allongée de l'élément de support, et étant adaptés pour s'engager dans deux évidements correspondants dans une paroi d'une cavité centrale d'un rotor de sorte que l'élément de support (41 ; 71 ; 91 ; 92 ; 111, 112 ; 131) s'étend à travers la cavité du rotor lorsque les prolongements latéraux sont engagés dans les évidements de celle-ci.

16. Ensemble de poches selon l'une quelconque des revendications 1 à 14, dans lequel :
l'élément de support comporte un corps allongé (131) ayant deux côtés latéraux opposés (A, B),
les moyens de montage comportent un premier et un second élément saillants (137, 138) s'étendant depuis un côté (A) du corps de l'élément de support (131), dans lequel les éléments saillants (137, 138) ont des pointes adaptées pour s'engager dans deux évidements dans une partie d'une paroi d'une cavité centrale d'un rotor de sorte que l'élément de support (131) s'étend sensiblement le long de la partie de paroi lorsque les éléments saillants (137, 138) sont engagés dans les évidements correspondants.

17. Ensemble de poches selon l'une quelconque des revendications 1 à 16, dans lequel les moyens de montage (43, 44, 73 ; 93, 94 ; 115, 116 ; 135, 136) et les premiers moyens de fixation (46, 47 ; 74 ; 95, 96, 97, 98 ; 119, 120 ; 137, 138) sont reliés à l'élément de support de sorte que la au moins une poche de produit (2, 3, 4, 26) est suspendue sensiblement dans une position verticale lorsque les moyens de montage montent le dispositif d'assemblage de poches (40 ; 70 ; 90 ; 110 ; 130) dans le rotor d'une centrifugeuse.

18. Ensemble de poches selon l'une quelconque des revendications 1 à 17, comportant en outre des seconds moyens de fixation (79, 80) reliés à l'élément de support (41 ; 71 ; 91 ; 92 ; 111 ; 112 ; 131) pour fixer de manière détachable la poche de séparation (1) à celui-ci.

19. Ensemble de poches selon la revendication 18, dans lequel les premiers moyens de fixation (46, 47 ; 74 ; 95, 96, 97, 98 ; 119, 120 ; 137, 138) et les seconds moyens de fixation (79, 80) sont reliés à l'élément de support de sorte que le tuyau de produit (15, 19, 23, 25) forme une boucle suffisamment grande pour empêcher le nouage de celui-ci lorsque la au moins une poche de produit (2,3, 4, 26) est fixée à l'élément de support (41 ; 71 ; 91 ; 92 ; 111 ; 112 ; 131) par les premiers moyens de fixation (46, 47 ; 74 ; 95, 96, 97, 98 ; 119, 120 ; 137, 138) et la poche de séparation (1) est fixée à l'élément de support (41 ; 71 ; 91 ; 92 ; 111 ; 112 ; 131) par les seconds moyens de fixation (79, 80).

20. Ensemble de poches selon la revendication 18 ou la revendication 19, dans lequel les seconds moyens de fixation (79, 80) sont adaptés pour fixer la poche de séparation (1) à l'élément de support (41 ; 71 ; 91 ; 92 ; 111 ; 112 ; 131) par un bord périphérique de celui-ci.

21. Ensemble de poches selon l'une quelconque des revendications 18 à 20, dans lequel la poche de séparation (1) est sensiblement annulaire ou semi annulaire et les seconds moyens de fixation (79, 80) sont adaptés pour fixer la poche de séparation (1) à l'élément de support (41 ; 71 ; 91 ; 92 ; 111 ; 112 ; 131) par un bord périphérique intérieur de celui-ci.

22. Ensemble de poches selon l'une quelconque des revendications 18 à 21, dans lequel les seconds moyens de fixation (79, 80) sont adaptés pour fixer la poche de séparation (1) à l'élément de support (41 ; 71 ; 91 ; 92 ; 111 ; 112 ; 131) par au moins deux zones correspondantes.

23. Ensemble de poches selon la revendication 22, dans lequel les seconds moyens de fixation comportent deux éléments saillants espacés (80) pour s'engager dans deux d'ouverture (11 ; 13) dans une zone périphérique de la poche de séparation (1).

24. Ensemble de poches selon la revendication 23, comportant en outre une poignée (77) reliée à l'élément de support (71) et les deux éléments saillants (80) s'étendent depuis la poignée (77).

25. Ensemble de poches selon la revendication 1, dans lequel les premiers moyens de fixation comportent deux éléments saillants espacés (46, 47 ; 95, 96, 97, 98 ; 119, 120 ; 137, 138) pour s'engager dans deux ouvertures (13) dans une zone périphérique de la poche de séparation (1) et deux trous (14) dans une zone périphérique de la au moins une poche de produit (2, 3, 4, 26).

26. Ensemble de poches selon l'une quelconque des revendications 1 à 25, comportant en outre une poche de collecte (29) reliée à la poche de séparation (1) par un tuyau de transfert (30).

27. Ensemble de poches selon la revendication 26, dans lequel le dispositif d'assemblage de poches (40 ; 70 ; 90 ; 110 ; 130) comporte en outre des troisième moyens de fixation (50, 51 ; 141, 142) reliée à l'élément de support (41 ; 71 ; 91 ; 92 ; 111 ; 112 ; 131) pour fixer de manière détachable la poche de collecte (29) à l'élément de support (41 ; 71 ; 91 ; 92 ; 111 ; 112 ; 131).

28. Ensemble de poches selon revendication 27, dans lequel l'élément de support comporte un corps allongé (41, 131) ayant un premier et un second côtés latéraux opposés (A, B), les premiers moyens de fixation comportent deux éléments saillants espacés (46, 47 ; 137, 138) pour s'engager dans deux trous (14) dans une zone périphérique de la au moins une poche de produit (2, 3, 4, 26), et les troisièmes moyens de fixation comportent deux éléments saillants espacés (50, 51 ; 141, 142) pour mettre en prise deux trous (14) dans une zone périphérique de la poche de collecte (29), les éléments saillants (46, 47 ; 137, 138) des premiers moyens de fixation s'étendant depuis le premier côté (A) du corps allongé (41, 131) et les éléments saillants (50, 51 ; 141, 142) des troisièmes moyens de fixation s'étendent depuis le second côté (B) du corps allongé (41, 131).

29. Ensemble de poches selon l'une quelconque des revendications 1 à 17, dans lequel les premiers moyens de fixation fixent de manière détachable la poche de séparation (1) à l'élément de support (41 ; 71 ; 91 ; 92 ; 111 ; 112 ; 131), et le dispositif d'assemblage de poches comporte en outre des troisième moyens de fixation (50, 51 ; 141, 142) reliés à l'élément de support (41 ; 71 ; 91 ; 92 ; 111 ; 112 ; 131) pour fixer de manière détachable la poche de collecte (29) à l'élément de support (41 ; 71 ; 91 ; 92 ; 111 ; 112 ; 131), en sorte que les premiers moyens de fixation (46, 47 ; 79, 80 ; 95, 96, 97, 98 ; 119, 120 ; 137, 138) et les troisièmes moyens de fixation (50, 51 ; 74 ; 95, 96, 97, 98 ; 119, 120 ; 141, 142) sont reliés à l'élément de support de sorte que le tuyau de transfert (30) forme une boucle suffisamment grande pour empêcher le nouage de celui-ci lorsque la poche de séparation (1) est fixée à l'élément de support (41 ; 71 ; 91 ; 92 ; 111 ; 112 ; 131) par les premiers moyens de fixation (46, 47 ; 74 ; 95, 96, 97, 98 ; 119, 120 ; 137, 138) et la poche de collecte (29) est fixée à l'élément de support (41 ; 71 ; 91 ; 92 ; 111 ; 112 ; 131) par les troisièmes moyens de fixation (50, 51 ; 74 ; 95, 96, 97, 98 ; 119, 120 ; 141, 142).

30. Ensemble de poches selon l'une quelconque des revendications 1 à 29, comportant en outre un filtre (27) relié à un tuyau (23 ; 25) relié à la au moins une poche de produit (4 ; 26).

31. Ensemble de poches selon la revendication 30, dans lequel le dispositif d'assemblage de poches (40 ; 90) comporte en outre un support (52 ; 103) relié à l'élément de support (41 ; 91) pour maintenir de manière détachable le filtre (27).

32. Ensemble de poches selon la revendication 31, dans lequel les moyens de montage (43, 44 ; 93, 94) et le porte-filtre (52 ; 103) sont reliés à l'élément de support de sorte que le porte-filtre (52 ; 103) est situé dans la cavité centrale d'un rotor lorsque le dispositif d'assemblage de poches (40 ; 90) est monté dans celui-ci.

33. Ensemble de poches selon la revendication 32, dans lequel le filtre (27) a un boîtier en forme de disque et le porte-filtre comporte une partie cylindrique (53, 105) pour contenir le filtre (27) de sorte que le filtre (27) et la partie cylindrique (53, 105) du support sont sensiblement concentriques.

34. Ensemble de poches selon revendication 33, dans lequel le porte-filtre est relié à l'élément de support (41 ; 91) de sorte qu'un axe central de la partie cylindrique (53, 103) du support est sensiblement perpendiculaire à un axe de rotation du rotor lorsque le dispositif d'assemblage de poches (40 ; 90) est monté dans un rotor.

35. Ensemble de poches selon la revendication la revendication 33 ou la revendication 34, dans lequel le porte-filtre (52, 103) et les premiers moyens de fixation (46, 47 ; 95, 96, 97, 98) sont reliés à l'élément de support de sorte que la au moins une poche de produit (2, 3, 4, 26) est sensiblement parallèle à un côté circulaire du boîtier en forme de disque du filtre (27).

36. Ensemble de poches selon l'une quelconque des revendications 30 à 35, dans lequel le dispositif d'assemblage de poches (40 ; 90) comporte en outre un premier support d'enroulement (53, 54, 105) pour enrouler une partie d'un tuyau (23 ; 25) relié au filtre.

37. Ensemble de poches selon l'une quelconque des revendications 1 à 36, dans lequel le dispositif d'assemblage de poches (40) comporte en outre un second support d'enroulement (50, 51, 54, 60, 61, 62) pour enrouler un tuyau de collecte (32).

38. Ensemble de poches selon l'une quelconque des revendications 1 à 37, comportant en outre une pince (16) pour fermer sélectivement le tuyau de produit (15, 19, 23) reliant la au moins une poche de produit (2, 3, 4) à la poche de séparation (1).
